# EUROPEAN PATENT APPLICATION

(11) **EP 2 093 297 A2**
(43) Date of publication of application: **26.08.2009**
(21) Application number: 09155512.8
(22) Date of filing: 31.08.2005
(51) Int. Cl.: C12Q 1/68, G01N 33/53

(54) **Agents for treatment or prevention of an allergic disorder**

(30) Priority: 07.09.2004 AU 2004905098; 07.09.2004 US 607270 P
(62) Divisional of application: 05775951.6
(71) Applicant: Telethon Institute for Child Health Research, Subiaco, Western Australia 6008 (AU)
(72) Inventor: Holt, Patrick, Nedlands Western Australia 6009 (AU); Sly, Peter, East Perth Western Australia 6004 (AU); Bosco, Anthony, Coogee Western Australia 6166 (AU); Devitt, Catherine, Claremont Western Australia 6010 (AU); McKenna, Katherine, Carine Western Australia 6020 (AU)
(74) Representative: Harrison Goddard Foote

(57) **Abstract**

The present invention relates to agents capable of treating and preventing an allergic disorder in an animal and a method of screening for these agents. In particular the invention relates to the NDFIP2 gene, its expression and agents that can modify its expression.

SEQUENCE LISTING

<110>  Telethon Institute for Child Health Research

<120>  Agents for Treatment or Prevention of an Allergic Disorder

<130>  FP22366

<140>  US60/607,270
<141>  2005-08-30

<150>  US60/607,270
<151>  2004-09-07

<150>  AU2004905098
<151>  2004-09-07

<160>  48

<170>  PatentIn version 3.3

<210>  1
<211>  21
<212>  DNA
<213>  Homo sapiens

<400>  1
caagaccggg gagaatacct g                                                21


<210>  2
<211>  23
<212>  DNA
<213>  Homo sapiens

<400>  2
gcgagaatgt ccaaatactc acc                                              23


<210>  3
<211>  21
<212>  DNA
<213>  Homo sapiens

<400>  3
gagtgaggtc accaagaatt c                                                21


<210>  4
<211>  26
<212>  DNA
<213>  Homo sapiens

<400>  4
cactctatct tctagatccc ttgaga                                           26


<210>  5
<211>  19
<212>  DNA
<213>  Homo sapiens

<400>  5
gcgtccctgg ccgtaattt                                                   19


<210>  6
<211>  20

<212>  DNA
<213>  Homo sapiens

<400>  6
tggttgctta gctggttgct                                                    20


<210>  7
<211>  21
<212>  DNA
<213>  Homo sapiens

<400>  7
aactcggtgt tcagtgagtg t                                                  21


<210>  8
<211>  19
<212>  DNA
<213>  Homo sapiens

<400>  8
ggagagggaa cggcaaact                                                     19


<210>  9
<211>  19
<212>  DNA
<213>  Homo sapiens

<400>  9
tgtggaggca cgaaaggat                                                     19


<210>  10
<211>  22
<212>  DNA
<213>  Homo sapiens

<400>  10
gatgggtaaa ccacaagaac ct                                                 22


<210>  11
<211>  21
<212>  DNA
<213>  Homo sapiens

<400>  11
tcaatctcgg ttggattcgg a                                                  21


<210>  12
<211>  23
<212>  DNA
<213>  Homo sapiens

<400>  12
ctgcttggta gagtgctgta agg                                                23


<210>  13
<211>  21
<212>  DNA
<213>  Homo sapiens

<400>  13
ttcaccaatg atcggcttca g                                                  21

<210>  14
<211>  21
<212>  DNA
<213>  Homo sapiens

<400>  14
ctccatctca tgctcaccaa g                                                21

<210>  15
<211>  22
<212>  DNA
<213>  Homo sapiens

<400>  15
tcgtgggtgc tgtgtttact ct                                               22

<210>  16
<211>  23
<212>  DNA
<213>  Homo sapiens

<400>  16
cagttggagg ttagacacag caa                                              23

<210>  17
<211>  20
<212>  DNA
<213>  Homo sapiens

<400>  17
cctgtctcag ccacgagcta                                                  20

<210>  18
<211>  23
<212>  DNA
<213>  Homo sapiens

<400>  18
tcctgaaaga tcatgtctgg taa                                              23

<210>  19
<211>  23
<212>  DNA
<213>  Homo sapiens

<400>  19
tcaatttaca cctgcgatca ctg                                              23

<210>  20
<211>  20
<212>  DNA
<213>  Homo sapiens

<400>  20
gttgtgggtc gtcatcacca                                                  20

<210>  21
<211>  26
<212>  DNA

<213>  Homo sapiens

<400>  21
agtccagata gtcgtatgtg aaatgc                                          26


<210>  22
<211>  25
<212>  DNA
<213>  Homo sapiens

<400>  22
ggtctgtcct tttatgacct caaac                                           25


<210>  23
<211>  19
<212>  DNA
<213>  Homo sapiens

<400>  23
cctgggcatg tgtcagagc                                                  19


<210>  24
<211>  22
<212>  DNA
<213>  Homo sapiens

<400>  24
ggtgttggag agtattgtgt gg                                              22


<210>  25
<211>  21
<212>  DNA
<213>  Homo sapiens

<400>  25
tcagcctcat cgacatatcc a                                               21


<210>  26
<211>  21
<212>  DNA
<213>  Homo sapiens

<400>  26
tcagccaatt caatgggctc c                                               21


<210>  27
<211>  24
<212>  DNA
<213>  Homo sapiens

<400>  27
gcagtcttac agcaattgaa cttt                                            24


<210>  28
<211>  21
<212>  DNA
<213>  Homo sapiens

<400>  28
ccatatcccc aaacttctga a                                               21

<210> 29
<211> 28
<212> DNA
<213> Homo sapiens

<400> 29
gtagatttag aagaagagaa ccaaaaac 28

<210> 30
<211> 20
<212> DNA
<213> Homo sapiens

<400> 30
cccaagcgct gtcttaactc 20

<210> 31
<211> 21
<212> DNA
<213> Homo sapiens

<400> 31
agtggggaat gatggcattt t 21

<210> 32
<211> 19
<212> DNA
<213> Homo sapiens

<400> 32
aaatccgcag atagcacca 19

<210> 33
<211> 21
<212> DNA
<213> Homo sapiens

<400> 33
cagaaactgg atgagccaac t 21

<210> 34
<211> 22
<212> DNA
<213> Homo sapiens

<400> 34
gacttccctc tgatctggta gg 22

<210> 35
<211> 22
<212> DNA
<213> Homo sapiens

<400> 35
tgcattgaca acgtactcag aa 22

<210> 36
<211> 22
<212> DNA
<213> Homo sapiens

<400> 36
tcatcttgac agggataagc at 22

<210> 37
<211> 21
<212> DNA
<213> Homo sapiens

<400> 37
gaacatcgac cgcatgaagg t 21

<210> 38
<211> 21
<212> DNA
<213> Homo sapiens

<400> 38
agaacacaaa agaggcgctt g 21

<210> 39
<211> 18
<212> DNA
<213> Homo sapiens

<400> 39
gcttcctccc gacgcaga 18

<210> 40
<211> 21
<212> DNA
<213> Homo sapiens

<400> 40
aacgaggatc ataatgcgaa a 21

<210> 41
<211> 26
<212> DNA
<213> Homo sapiens

<400> 41
tcttatttca ctttctcaac tcatca 26

<210> 42
<211> 25
<212> DNA
<213> Homo sapiens

<400> 42
ggcataacct gaatgtataa ttcaa 25

<210> 43
<211> 26
<212> DNA
<213> Homo sapiens

<400> 43
gaaaaagctg ttgagtgaag aagact 26

<210> 44
<211> 22
<212> DNA
<213> Homo sapiens

<400> 44
tgcaggatga gcaatgctga ga 22

<210> 45
<211> 20
<212> DNA
<213> Homo sapiens

<400> 45
gggaatctgg ctggtattgg 20

<210> 46
<211> 23
<212> DNA
<213> Homo sapiens

<400> 46
ttctggcatc ttctgcaggt gtt 23

<210> 47
<211> 24
<212> DNA
<213> Homo sapiens

<400> 47
aacagcctca cagagcagaa gact 24

<210> 48
<211> 22
<212> DNA
<213> Homo sapiens

<400> 48
cagcgagtgt ccttctcatg gt 22

## Description

### FIELD OF THE INVENTION

The present invention relates to agents capable of treating or preventing an allergic disorder in an animal and a method of screening for these agents. The invention provides genes whose expression is modulated differently in allergic subjects compared to non-allergic subjects. Some of these genes are novel *per se*, while others are known, but have not previously been associated with allergic conditions.

In one embodiment, the invention relates to a method of screening for an agent capable of treating or preventing an allergic disorder such as asthma by identifying mRNA transcripts from specific allergy-associated genes or protein encoded by the mRNA in a biological sample, exposing the biological sample to a test agent, and determining whether the level of mRNA or the corresponding protein in the sample changes following the contacting step.

### BACKGROUND OF THE INVENTION

All references, including any patents or patent applications, cited in this specification are hereby incorporated by reference. No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents forms part of the common general knowledge in the art, in Australia or in any other country.
Allergic disorders such as asthma, atopic dermatitis, hyper-IgE syndrome, and allergic rhinitis represent some of the commonest and best-characterised immune disorders in humans. Indeed, allergic disorders affect roughly 20% of the population of the United States.

Current treatment of allergic disorders includes allergen avoidance, pharmaceutical-based therapy and immunotherapy. Completely avoiding allergen exposure is the most logical approach, but this is very difficult or impossible to achieve in the vast majority of cases. Pharmaceuticals such as antihistamines, steroids, beta-agonists and adrenaline are useful, but they only alleviate the symptoms of allergy without influencing its causes. In addition, pharmaceutical treatment is usually limited by undesirable side effects, particularly in the case of steroids.

Current immunotherapeutic approaches include desensitisation, allergen alteration aiming at reducing recognition by specific antibodies, and the use of allergen-derived peptides, which interfere in the cognate interaction between specific B and T cells. Desensitisation therapy involves repeated injections with increasing dosages of a crude allergen extract of the offending allergen. Although treatment with allergen extracts has been proven reasonably effective in the clinic for alleviating allergen-related symptoms, and is a common therapy used widely in allergy clinics today, the mechanism of desensitisation remains unclear. Furthermore, desensitisation therapy must be undertaken with extreme caution, as anaphylactic side effects may be significant or even fatal.

Accordingly, although all of the above therapies have a role to play in the treatment of allergic disorders at present there is still a need in the art for more effective treatments. Unfortunately, the development of these treatments has been hampered by the lack of understanding about the aetiology of allergy, which has still to be elucidated.

The inventors believe that they have developed a greater understanding of the mechanisms underlying allergy, which has enabled them to develop a more effective method of therapy and method of screening for agents capable of preventing and/or treating allergic disorders in animals such as humans.

### SUMMARY OF THE INVENTION

The inventors have found that the degree of expression of a number of genes, including some which are novel and others which are known, but which had not previously been suggested to be associated with allergic disorders, is modulated in allergen-stimulated peripheral blood mononuclear cells (PMBC) from animals suffering from an allergic disorder. However, the expression of these genes is modulated in a different way or is unmodulated in animals which do not have an allergic disorder. In most cases the observed modulation was upregulation or activation of expression; however, in some cases the modulation was down regulation of expression.

In a first aspect the invention provides
(a) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively,
(b) an isolated nucleic acid molecule which is the complement of the nucleic acid of (a), or
(c) an isolated nucleic acid molecule which hybridizes under stringent conditions to the nucleic acid of (a)or (b).

This aspect of the invention also provides an isolated polypeptide molecule encoded by the nucleic acid of (a), (b) or (c).

In a second aspect the invention provides a therapeutic or prophylactic composition comprising
(i) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of cig5, IFIT4, LAMP3, DACT1, KRT1, LNPEP, NCOA3, OAZ, PECAM1, PLXDC1, RASGRP3, SLC39A8, RAB27B, GNG8 and GJB2, or in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or
(ii) an isolated nucleic acid molecule which is the complement of the nucleic acid of (a),
(iii) an isolated nucleic acid molecule which hybridizes under stringent conditions to the nucleic acid of (a)or (b), or
(iv) an isolated polypeptide molecule encoded by the nucleic acid of (a), (b) or (c).

In a third aspect the invention provides
(i) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively,
(ii) an isolated nucleic acid molecule which is the complement of the nucleic acid of (a), or
(iii) an isolated nucleic acid molecule which hybridizes under stringent conditions to the nucleic acid of (a) or (b), or
(iv) an isolated polypeptide molecule encoded by the nucleic acid of (a), (b) or (c), for use in the therapy or prophylaxis of an allergic condition.

In a fourth aspect the invention provides a primer set for amplification of a nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the primer set is selected from the group consisting of
cig5 forward: 5'CAAGACCGGGGAGAATACCTG3' (SEQ ID NO:1)
cig5 reverse: 5'GCGAGAATGTCCAAATACTCACC3' (SEQ ID NO:2)
IFIT4 forward: 5'GAGTGAGGTCACCAAGAATTC3' (SEQ ID NO:3)
IFIT4 reverse: 5'CACTCTATCTTCTAGATCCCTTGAGA3' (SEQ ID NO:4)
LAMP3 forward: 5'GCGTCCCTGGCCGTAATTT3' (SEQ ID NO:5)
LAMP3 reverse: 5'TGGTTGCTTAGCTGGTTGCT3' (SEQ ID NO:6)
DACT1 forward: 5'AACTCGGTGTTCAGTGAGTGT3' (SEQ ID NO:7)
DACT1 reverse: 5'GGAGAGGGAACGGCAAACT3' (SEQ ID NO:8)
IL17RB forward: 5'TGTGGAGGCACGAAAGGAT3' (SEQ ID NO:9)
IL17RB reverse: GATGGGTAAACCACAAGAACCT3' (SEQ ID NO:10)
KRT1 forward: 5'TCAATCTCGGTTGGATTCGGA3' (SEQ ID NO:11)
KRT1 reverse: 5'CTGCTTGGTAGAGTGCTGTAAGG3' (SEQ ID NO:12)
LNPEP forward: 5'TTCACCAATGATCGGCTTCAG3' (SEQ ID NO:13)
LNPEP reverse: 5'CTCCATCTCATGCTCACCAAG3' (SEQ ID NO:14)
MAL forward: 5'TCGTGGGTGCTGTGTTTACTCT3' (SEQ ID NO:15)
MAL reverse: 5' CAGTTGGAGGTTAGACACAGCAA3' (SEQ ID NO:16)
NCOA3 forward: 5'CCTGTCTCAGCCACGAGCTA3' (SEQ ID NO:17)
NCOA3 reverse: 5'TCCTGAAAGATCATGTCTGGTAA3' (SEQ ID NO:18)
OAZ forward: 5'TCAATTTACACCTGCGATCACTG3' (SEQ ID NO:19)
OAZ reverse: 5'GTTGTGGGTCGTCATCACCA3' (SEQ ID NO:20)
PECAM1 forward: 5'AGTCCAGATAGTCGTATGTGAAATGC3' (SEQ ID NO:21)
PECAM1 reverse: GGTCTGTCCTTTTATGACCTCAAAC3' (SEQ ID NO:22)
PLXDC1 forward: 5'CCTGGGCATGTGTCAGAGC3' (SEQ ID NO:23)
PLXDC1 reverse: 5'GGTGTTGGAGAGTATTGTGTGG3' (SEQ ID NO:24)
RASGRP3 forward: 5'TCAGCCTCATCGACATATCCA3' (SEQ ID NO:25)
RASGRP3 reverse: 5'TCAGCCAATTCAATGGGCTCC3' (SEQ ID NO:26)
SLC39A8 forward: 5'GCAGTCTTACAGCAATTGAACTTT3' (SEQ ID NO:27)
SLC39A8 reverse: 5'CCATATCCCCAAACTTCTGAA3' (SEQ ID NO:28)
XBP1 forward: 5'GTAGATTTAGAAGAAGAGAACCAAAAAC3' (SEQ ID NO:29)
XBP1 reverse: 5'CCCAAGCGCTGTCTTAACTC3' (SEQ ID NO:30)
NDFIP2 forward: 5'AGTGGGGAATGATGGCATTTT3' (SEQ ID NO:31)
NDFIP2 reverse: AAATCCGCAGATAGCACCA3' (SEQ ID NO:32)
RAB27B forward: 5'CAGAAACTGGATGAGCCAACT3' (SEQ ID NO:33)
RAB27B reverse: 5'GACTTCCCTCTGATCTGGTAGG3' (SEQ ID NO:34)
243610_at forward: 5'TGCATTGACAACGTACTCAGAA3' (SEQ ID NO:35)
243610_at reverse: 5'TCATCTTGACAGGGATAAGCAT3' (SEQ ID NO:36)
GNG8 forward: 5'GAACATCGACCGCATGAAGGT3' (SEQ ID NO:37)
GNG8 reverse: 5'AGAACACAAAAGAGGCGCTTG3' (SEQ ID NO:38)
GJB2 forward: 5'GCTTCCTCCCGACGCAGA3' (SEQ ID NO:39)
GJB2 reverse: 5'AACGAGGATCATAATGCGAAA3' (SEQ ID NO:40)
1556097_at forward: 5'TCTTATTTCACTTTCTCAACTCATCA3' (SEQ ID NO:41)
1556097_at reverse: 5'GGCATAACCTGAATGTATAATTCAA3' (SEQ ID NO:42)
242743_at forward: 5'GAAAAAGCTGTTGAGTGAAGAAGACT3' (SEQ ID NO:43)
242743_at reverse: 5'TGCAGGATGAGCAATGCTGAGA3' (SEQ ID NO:44)
and
CISH forward: 5'GGGAATCTGGCTGGTATTGG3' (SEQ ID NO:45)
CISH reverse: 5'TTCTGGCATCTTCTGCAGGTGTT3' (SEQ ID NO:46).

In a fifth aspect the invention provides a kit for screening for an agent capable of treating or preventing an allergic disorder in a mammal, comprising one or more primers specific for a region of at least one gene which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively.

In a sixth aspect the invention provides a method of treating or preventing an allergic disorder in a mammal, comprising the step of administering to the mammal a therapeutic or prophylactic agent in an amount sufficient to regulate the expression of a gene which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or a combination of two or more of these genes.

In some embodiments the agent is a down-regulator of expression of one or more genes selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or a combination of two or more of these genes.

In other embodiments the agent is an up-regulator of expression of cig5, IFIT4, or LAMP3.

The agent may be administered in the form of a composition further comprising a pharmaceutically acceptable carrier. This will usually comprise at least one excipient, for example selected from the group consisting of sterile water, sodium phosphate, mannitol, sorbitol, sodium chloride, and any combination thereof.

In a seventh aspect the invention provides a method of screening for an agent capable of treating or preventing an allergic disorder in a mammal, the method comprising:
(a) providing a biological sample from a mammal;
(b) determining the degree of activation of one or more specific genes in the sample,
   wherein the gene is associated with an allergic disorder;
(c) contacting the biological sample with a candidate agent; and
(d) determining whether the degree of activation changes in the sample following the contacting step,
wherein a change in the degree of activation observed in the presence of the agent compared to that observed in the absence of the agent indicates that the agent is capable of treating or preventing an allergic disorder.

In an eighth aspect the invention provides a method of screening for an agent capable of treating or preventing an allergic disorder in a mammal, the method comprising:
(a) providing a biological sample from the mammal;
(b) determining the level of mRNA transcripts from one or more allergy-associated genes,
   in which the gene is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively;
(c) contacting the biological sample with the agent; and
(d) determining whether the level of mRNA transcripts changes in the sample following the contacting step,
in which the change in the level of mRNA transcripts in the sample following contact with the agent compared to in the absence of the agent indicates that the agent is capable of treating or preventing an allergic disorder.

In a ninth aspect, the invention provides a method of selecting an appropriate therapeutic agent for treatment of a mammal which suffers from or is predisposed to developing an allergic disorder, comprising the steps of:
(a) obtaining a biological sample from the mammal;
(b) determining the level in the sample of mRNA transcripts from one or more genes which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or combination thereof; and
(c) selecting a therapeutic modulator which compensates for the presence or absence of the mRNA transcript or protein.

In some embodiments, the modulator is an agonist, while in an alternative embodiment, the modulator is an antagonist. In the seventh to the ninth aspects of the invention the gene may be any gene associated with an allergic disorder. Preferably the gene is one or more selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively and may be a combination of two or more of these genes.

In all aspects of the invention the step of determining the level of activation of the gene can be performed by any method known in the art. Preferably this is carried out by detecting the presence of mRNA by reverse transcription polymerase chain reaction (RT-PCR), or using specific nucleic acid arrays utilising microchip technology. Alternatively, the level of activation is determined by the detection of the protein encoded by the mRNA, for example using ELISA, proteomic arrays, or intracellular staining as detected by flow cytometry. All of these methods are well known in the art. It will be appreciated that in some cases the gene will be inactive, i.e. will not be transcribed or translated to a significant extent, while in other cases the expression of the gene will be modulated, i.e. it will be upregulated or down regulated.

For all aspects of the invention, in one embodiment the allergy-associated gene is upregulated in allergen-challenged PBMC from atopic individuals but is upregulated weakly if at all in PBMC from individuals who are not allergic to that allergen. Preferably in this embodiment the gene is one or more selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, and genes which comprise a sequence selected from the group consisting of sequences identified by probes 243610_at on human chromosome 9q21.13 at locus 138255, 1556097_at on human chromosome 15q25.2 and 242743_at on human chromosome 16p12.1 respectively. More preferably the gene is upregulated in atopic individuals and down-regulated in non-atopic individuals. Even more preferably the gene is KRT₁, PECAM₁ or PLXDC₁.

In alternative embodiments the allergy-associated gene is down-regulated in allergen-challenged PBMC from non-atopic individuals, but is not down-regulated in PBMC from atopic individuals. Preferably in these embodiments the gene is selected from the group consisting of cig5, IFIT4 and LAMP3.

The biological sample can be any biological material isolated from an atopic or non-atopic mammal, including but not limited to blood, bone marrow, plasma, serum, lymph, cerebrospinal fluid, or a cellular or fluid component thereof; external sections of the skin, respiratory, intestinal, and genitourinary tracts; other secretions such as tears, saliva, or milk; tissue or organ biopsy samples; or cultured cells or cell culture supernatants. Preferably the biological sample is blood or lymph, or a cellular or fluid component thereof. More preferably the biological sample is bone marrow-derived mononuclear cells from peripheral blood (PBMC), which have been stimulated by *in vitro* exposure to one or more allergens to which the mammal is allergic. The skilled person will readily be able to determine whether prior exposure to allergen *in vitro* is advantageous in a particular case. This may depend on the nature of the biological sample.

In some embodiments of the sixth to the ninth aspects of the invention the agent is an siRNA molecule or an anti-sense oligonucleotide.

Preferably the agent is an anti-sense oligonucleotide of 8 to 50 nucleotide bases in length, which specifically hybridizes with a coding region of a gene which is selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 or CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, and inhibits the expression of the gene.

In other embodiments the agent is either a polyclonal or monoclonal antibody. Preferably the antibody is a humanized monoclonal antibody.

In yet other embodiments the agent is a steroid, a β-2 agonist, a methylxanthine, a leukotriene modifier, an anti-cholinergic, a systemic corticosteroid, or an anti-histamine.

The mammal may be a human, or may be a domestic, companion or zoo animal. While it is particularly contemplated that the compounds of the invention are suitable for use in medical treatment of humans, they are also applicable to veterinary treatment, including treatment of companion animals such as dogs and cats, and domestic animals such as horses, cattle and sheep, or zoo animals such as non-human primates, felids, canids, bovids, and ungulates.
Methods and pharmaceutical carriers for preparation of pharmaceutical compositions are well known in the art, as set out in textbooks such as Remington's Pharmaceutical Sciences, 20th Edition, Williams & Wilkins, Pennsylvania, USA.

The compounds and compositions of the invention may be administered by any suitable route, and the person skilled in the art will readily be able to determine the most suitable route and dose for the condition to be treated. Dosage will be at the discretion of the attendant physician or veterinarian, and will depend on the nature and state of the condition to be treated, the age and general state of health of the subject to be treated, the route of administration, and any previous treatment which may have been administered.

The carrier or diluent, and other excipients, will depend on the route of administration, and again the person skilled in the art will readily be able to determine the most suitable formulation for each particular case.

### BRIEF DESCRIPTION OF THE FIGURES

Figure 1 shows the results of the CD69, CFSE, CD4 and CD8 kinetic experiments performed on HGU133A arrays, and the PMBC kinetic experiment performed on HGU133plus2 arrays. t16, t24 and t48 represent 16, 24 and 48 hours of culture.
Figure 2 shows a comparison of the level of expression of the gene cig5 in CD4 cells from individuals allergic to HDM and from non-allergic individuals.
Figure 3 shows a comparison of the level of expression of the gene IFIT4 in CD4 cells from individuals allergic to HDM and from non-allergic individuals.
Figure 4 shows a comparison of the level of expression of the gene LAMP3 in CD4 cells from individuals allergic to HDM and from non-allergic individuals.
Figure 5 shows a comparison of the level of expression of the gene DACT1 in CD4 cells from individuals allergic to HDM and from non-allergic individuals.
Figure 6 shows a comparison of the level of expression of the gene IL17RB in CD4 cells from individuals allergic to HDM and from non-allergic individuals.
Figure 7 shows a comparison of the level of expression of the gene KRT1 in CD4 cells from individuals allergic to HDM and from non-allergic individuals.
Figure 8 shows a comparison of the level of expression of the gene LNPEP expression in CD4 cells from individuals allergic to HDM and from non-allergic individuals.
Figure 9 shows a comparison of the level of expression of the gene MAL in CD4 cells from individuals allergic to HDM and from non-allergic individuals.
Figure 10 shows a comparison of the level of expression of the gene NCOA3 in CD4 cells from individuals allergic to HDM and from non-allergic individuals.
Figure 11 shows a comparison of the level of expression of the gene OAZ in CD4 cells from individuals allergic to HDM and from non-allergic individuals.
Figure 12 shows a comparison of the level of expression of the gene PECAM1 in CD4 cells from individuals allergic to HDM and from non-allergic individuals.
Figure 13 shows a comparison of the level of expression of the gene PLXDC1 in CD4 cells from individuals allergic to HDM and from non-allergic individuals.
Figure 14 shows a comparison of the level of expression of the gene RASGRP3 in CD4 cells from individuals allergic to HDM and from non-allergic individuals.
Figure 15 shows a comparison of the level of expression of the gene SLC39A8 in CD4 cells from individuals allergic to HDM and from non-allergic individuals.
Figure 16 shows a comparison of the level of expression of the gene XBP1 in CD4 cells from individuals allergic to HDM and from non-allergic individuals.
Figure 17 shows a comparison of the level of expression of the gene CISH in CD4 cells from individuals allergic to HDM and from non-allergic individuals.
Figure 18 shows a comparison of the level of expression of the gene cig5 in PBMC from individuals allergic to HDM and from non-allergic individuals.
Figure 19 shows a comparison of the level of expression of the gene IFIT4 in PBMC from individuals allergic to HDM and from non-allergic individuals.
Figure 20 shows a comparison of the level of expression of the gene LAMP3 in PBMC from individuals allergic to HDM and from non-allergic individuals.
Figure 21 shows a comparison of the level of expression of the gene DACT1 in PBMC from individuals allergic to HDM and from non-allergic individuals.
Figure 22 shows a comparison of the level of expression of the gene IL17RB in PBMC from individuals allergic to HDM and from non-allergic individuals.
Figure 23 shows a comparison of the level of expression of the gene KRT1 expression in PBMC from individuals allergic to HDM and from non-allergic individuals.
Figure 24 shows a comparison of the level of expression of the gene LNPEP expression in PBMC from individuals allergic to HDM and from non-allergic individuals.
Figure 25 shows a comparison of the level of expression of the gene MAL expression in PBMC from individuals allergic to HDM and from non-allergic individuals.
Figure 26 shows a comparison of the level of expression of the gene NCOA3 expression in PBMC from individuals allergic to HDM and from non-allergic individuals.
Figure 27 shows a comparison of the level of expression of the gene OAZ expression in PBMC from individuals allergic to HDM and from non-allergic individuals.
Figure 28 shows a comparison of the level of expression of the gene PECAM1 expression in PBMC from individuals allergic to HDM and from non-allergic individuals.
Figure 29 shows a comparison of the level of expression of the gene PLXDC1 expression in PBMC from individuals allergic to HDM and from non-allergic individuals.
Figure 30 shows a comparison of the level of expression of the gene RASGRP3 expression in PBMC from individuals allergic to HDM and from non-allergic individuals.
Figure 31 shows a comparison of the level of expression of the gene SLC39A8 expression in PBMC from individuals allergic to HDM and from non-allergic individuals.
Figure 32 shows a comparison of the level of expression of the gene XBP1 expression in PBMC from individuals allergic to HDM and from non-allergic individuals.
Figure 33 shows a comparison of the level of expression of the gene NDFIP2 expression in PBMC from individuals allergic to HDM and from non-allergic individuals.
Figure 34 shows a comparison of the level of expression of the gene RAB27B expression in PBMC from individuals allergic to HDM and from non-allergic individuals.
Figure 35 shows a comparison of the level of expression of the gene 242743_AT expression in PBMC from individuals allergic to HDM and from non-allergic individuals.
Figure 36 shows a comparison of the level of expression of the gene GNG8 expression in PBMC from individuals allergic to HDM and from non-allergic individuals.
Figure 37 shows a comparison of the level of expression of the gene GJB2 expression in PBMC from individuals allergic to HDM and from non-allergic individuals.
Figure 38 shows a comparison of the level of expression of the gene 1556097 expression in PBMC from individuals allergic to HDM and from non-allergic individuals.
Figure 39 shows a comparison of the level of expression of the gene 243610_AT expression in PBMC from individuals allergic to HDM and from non-allergic individuals.
Figure 40 shows a comparison of the level of expression of the gene CISH expression in PBMC from individuals allergic to HDM and from non-allergic individuals.
Figure 41 shows a comparison of the level of expression of IL4 mRNA expression at 16 hours post-stimulation for CD4+ cells from individuals allergic to HDM and from non-allergic individuals as assessed by quantitative real-time PCR.
Figure 42 shows a comparison of the level of expression of DACT1 mRNA expression at 16 hours post-stimulation for CD4+ cells from individuals allergic to HDM and from non-allergic individuals as assessed by quantitative real-time PCR.
Figure 43 shows a comparison of the level of expression of LAMP3 mRNA expression at 16 hours post-stimulation for CD4+ cells from individuals allergic to HDM and from non-allergic individuals as assessed by quantitative real-time PCR.
Figure 44 shows a comparison of the level of expression of PLXDC1 mRNA expression at 16 hours post-stimulation for CD4+ cells from individuals allergic to HDM and from non-allergic individuals as assessed by quantitative real-time PCR.
Figure 45 shows a comparison of the level of expression of PLXDC1 mRNA expression at 48 hours post-stimulation for CD4+ cells from individuals allergic to HDM and from non-allergic individuals as assessed by quantitative real-time PCR.
Figure 46 shows a comparison of the level of expression of cig5 mRNA expression at 16 hours post-stimulation for CD4+ cells from individuals allergic to HDM and from non-allergic individuals as assessed by quantitative real-time PCR.
Figure 47 shows a comparison of the level of expression of IFIT4 mRNA expression at 16 hours post-stimulation for CD4+ cells from individuals allergic to HDM and from non-allergic individuals as assessed by quantitative real-time PCR.
Figure 48 shows a comparison of the level of expression of MAL mRNA expression at 16 hours post-stimulation for CD4+ cells from individuals allergic to HDM and from non-allergic individuals as assessed by quantitative real-time PCR.
Figure 49 shows a comparison of the level of expression of PECAM1 mRNA expression at 16 hours post-stimulation for CD4+ cells from individuals allergic to HDM and from non-allergic individuals as assessed by quantitative real-time PCR.
Figure 50 shows a comparison of the level of expression of SLC39A8 mRNA expression at 16 hours post-stimulation for CD4+ cells from individuals allergic to HDM and from non-allergic individuals as assessed by quantitative real-time PCR.
Figure 51 shows a comparison of the level of expression of XBP1 mRNA expression at 16 hours post-stimulation for CD4+ cells from individuals allergic to HDM and from non-allergic individuals as assessed by quantitative real-time PCR.
Figure 52 shows a comparison of the level of expression of NDFIP2 mRNA expression at 16 hours post-stimulation for CD4+ cells from individuals allergic to HDM and from non-allergic individuals as assessed by quantitative real-time PCR.
Figure 53 shows a comparison of the level of expression of 243610_at CISH mRNA expression at 16 hours post-stimulation for CD4+ cells from individuals allergic to HDM and from non-allergic individuals as assessed by quantitative real-time PCR.
Figure 54 shows a comparison of the level of expression of CISH mRNA expression at 16 hours post-stimulation for CD4+ cells from individuals allergic to HDM and from non-allergic individuals as assessed by quantitative real-time PCR.
Figure 55 shows a comparison of the level of expression of NCOA3 mRNA expression at 48 hours post-stimulation for CD4+ cells from individuals allergic to HDM and from non-allergic individuals as assessed by quantitative real-time PCR.
Figure 56 shows a comparison of the level of expression of NDFIP2 mRNA expression at 16 hours post-stimulation for PBMC cells from individuals allergic to HDM and from non-allergic individuals as assessed by quantitative real-time PCR.
Figure 57 shows a comparison of the level of expression of RAB27B mRNA expression at 16 hours post-stimulation for PBMC cells from individuals allergic to HDM and from non-allergic individuals as assessed by quantitative real-time PCR.
Figure 58 shows a comparison of the level of expression of 243610_at mRNA expression at 16 hours post-stimulation for PBMC cells from individuals allergic to HDM and from non-allergic individuals as assessed by quantitative real-time PCR.
Figure 59 shows a comparison of the level of expression of GNG8 mRNA expression at 16 hours post-stimulation for PBMC cells from individuals allergic to HDM and from non-allergic individuals as assessed by quantitative real-time PCR.
Figure 60 shows a comparison of the level of expression of GJB2 mRNA expression at 16 hours post-stimulation for PBMC cells from individuals allergic to HDM and from non-allergic individuals as assessed by quantitative real-time PCR.
Figure 61 shows a comparison of the level of expression of 1556097_at mRNA expression at 16 hours post-stimulation for PBMC cells from individuals allergic to HDM and from non-allergic individuals as assessed by quantitative real-time PCR.
Figure 62 shows a comparison of the level of expression of 242743_at mRNA expression at 16 hours post-stimulation for CD4⁺ cells from individuals allergic to HDM and from non-allergic individuals as assessed by quantitative real-time PCR.
Figure 63 shows a comparison of the level of expression of 242743_at mRNA expression at 16 hours post-stimulation for PBMC cells from individuals allergic to HDM and from non-allergic individuals as assessed by quantitative real-time PCR.

### DETAILED DESCRIPTION OF THE INVENTION

Before describing the present invention in detail, it is to be understood that this invention is not limited to particularly exemplified methods of diagnosis and may, of course, vary. It is also to be understood that the terminology used herein is for the purpose of describing particular embodiments of the invention only, and is not intended to be limiting which will be limited only by the appended claims.

All publications, patents and patent applications cited herein, whether *supra* or *infra*, are hereby incorporated by reference in their entirety. However, publications mentioned herein are cited for the purpose of describing and disclosing the protocols and reagents which are reported in the publications and which might be used in connection with the invention. Nothing herein is to be construed as an admission that the invention is not entitled to antedate such a disclosure by virtue of prior invention.

Furthermore, the practice of the present invention employs, unless otherwise indicated, conventional immunological, chemistry and pharmacology techniques within the skill of the art. Such techniques are well known to the skilled worker, and are explained fully in the literature. See, e.g., Coligan, Dunn, Ploegh, Speicher and Wingfield "Current protocols in Protein Science" (1999) Volume I and II (John Wiley & Sons Inc.); and Bailey, J.E. and Ollis, D.F., Biochemical Engineering Fundamentals, McGraw-Hill Book Company, NY, 1986.

### Abbreviations used herein are as follows:

- ELISA: enzyme-linked immunoadsorbent assay
- HDM: house dust mite
- IL-4: interleukin 4
- PCR: polymerase chain reaction
- PBMC: peripheral blood mononuclear cells
- RT-PCR: reverse transcriptase polymerase chain reaction.

In the claims of this application and in the description of the invention, except where the context requires otherwise due to express language or necessary implication, the words "comprise" or variations such as "comprises" or "comprising" are used in an inclusive sense, i.e. to specify the presence of the stated features but not to preclude the presence or addition of further features in various embodiments of the invention.
As used herein and in the appended claims, the singular forms "a," "an," and "the" include plural reference unless the context clearly dictates otherwise. Thus, for example, a reference to "a gene" includes a plurality of such genes, and a reference to "an allergy" is a reference to one or more allergies, and so forth.

Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.
Where a range of values is expressed, it will be clearly understood that this range encompasses the upper and lower limits of the range, and all values in between these limits.

Without wishing to be bound by any particular theory or hypothesis, the inventors have observed and demonstrated that a number of genes, including some which had not previously been considered to be associated with allergic disorders, are activated in allergen-stimulated PMBC from animals which have an allergic disorder. However, these genes are activated to a lesser extent, if at all, in animals which do not have an allergic disorder. For example, the inventors have found that genes such as DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, and genes which comprise a sequence selected from the group consisting of sequences identified by probes 243610_at on human chromosome 9q21.13 at locus 138255, 1556097_at on human chromosome 15q25.2 and 242743__at on human chromosome 16p12.1 respectively, are strongly activated in house dust mite (HDM)-stimulated PMBC from humans allergic to house dust mite, whereas these genes are activated only weakly or not at all in PBMC from humans not allergic to house dust mite.
In contrast, other genes such as cig5, IFIT4 and LAMP3 are actively down-regulated in HDM-stimulated PBMC from non-atopic individuals (normal individuals), whereas these genes are not down-regulated in corresponding PBMC samples from atopic ("allergic") individuals. These genes are still considered to be indicative of the non-atopic phenotype; they are also considered to be representative of "protective" genes i.e. the products of these genes in some way provides protection from the development of allergy.

Accordingly, in some embodiments, the terms "allergy-associated genes" or "allergy-specific genes", which are used herein interchangeably, refer to genes which are either typically associated with an allergic disorder, or are shown to be associated with an allergic disorder, in that an animal exhibiting clinical symptoms of an allergic disorder has a gene which is activated in the presence of a stimulating compound or allergen, and the level of activation of the gene is different to that in a non-allergic animal. Activation of the gene will normally be demonstrated by expression in vitro of cells from an allergic animal in response to the allergen.

The term "activated" as used herein means that the gene is actively being transcribed in an animal, i.e. the corresponding mRNA or the protein encoded by that mRNA can be detected.

As used herein, the terms "allergic disorder" or "allergic condition" refers to an abnormal biological function characterised by either an increased responsiveness of the trachea and bronchi to various stimuli or by a disorder involving inflammation at these or other sites in response to allergen exposure. The symptoms associated with these allergic disorders include, but are not limited to, cold, cold-like, and/or "flu-like" symptoms, cough, dermal irritation, dyspnea, lacrimation, rhinorrhea, sneezing and wheezing, and skin manifestations. Allergic disorders are also often associated with an increase in Th2 cytokines such as IL-4, IL-4R, IL-5, IL-9 and IL-13. Examples of allergic disorders include, but are not limited to, asthma, atopic dermatitis, bronchoconstriction, chronic airway inflammation, allergic contact dermatitis, eczema, food allergy, hay fever, hyper-IgE syndrome, rhinitis, and allergic urticaria.

The term "mammal" as used herein includes, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs. The terms do not denote a particular age, and thus both adult and immature individuals are intended to be covered. The methods described herein are intended for use in any of the above mammalian species, since the immune systems of all of these mammals operate similarly.

Thus the invention encompasses the screening for agents capable of treating or preventing an allergic disorder in any mammal, including a human, as well as those mammals of economic and/or social importance to humans, including carnivores such as cats, dogs and larger felids and canids, swine such as pigs, hogs, and wild boars, ruminants such as cattle, oxen, sheep, giraffes, deer, goats, bison, and camels, and horses, and non-human primates such as apes and monkeys. Thus the invention encompasses the treatment of livestock, including, but not limited to, domesticated swine, ruminants, horses, and the like, and zoo or endangered animals.

As used herein, the term "atopic" or "allergic" refers to an animal which has an allergic reaction. Conversely a "non-atopic" animal is one which does not have an allergic reaction. Allergy is conventionally diagnosed by skin tests such as the skin prick, intradermal or skin patch test, by determination of IgE antibody radioallergosorbent testing (RAST), or by ELISA or related methods.
Allergies are caused by allergens, which may be present in a wide variety of sources, including but not limited to pollens or other plant components, dust, moulds or fungi, foods, animal or bird danders, insect venoms, or chemicals.

The biological sample may be tested using the techniques described herein directly after isolation, or may be further processed in order to increase the quality of the data produced. In this regard, the inventors have noted from the literature that the selective expansion of allergen-specific cells by initial stimulation with allergen to induce proliferation generates a "cell line" in which the frequency of the relevant cells is a log scale greater than the same cells in a biological sample directly isolated from an animal is useful. If required, the cells can be further concentrated and purified by cloning the specific cells, using known methods.

Accordingly, in one embodiment, a biological sample such as peripheral blood is taken from an animal which is suspected of, or susceptible to the development of an allergic disorder. The biological sample is then treated so as to substantially isolate leukocytes from the blood i.e. separate the leukocytes from (or otherwise substantially free from), other contaminant cells. The biological sample is then exposed to an environmental allergen. The term "environmental allergen" as used herein refers to allergens which are specifically associated with the development of allergic disorders. For example, such allergens include those of animals, including the house dust mite, e.g. *Dermatophagoides pteronyssinus, Dermatophagoides farinae*, *Biomia tropicalis*, such as the house dust mite allergens der p1 (Scobie et al. (1994) Biochem. Soc. Trans. 22: 448S; Yssel et al. (1992) J. Immunol. 148: 738-745), der p2 (Chua et al. (1996) Clin. Exp. Allergy 26: 829-837), der p3 (Smith and Thomas (1996) Clin. Exp. Allergy 26: 571-579), der p5, der p V (Lin et al. (1994) J. Allergy Clin. Immunol. 94: 989-996), der p6 (Bennett & Thomas (1996) Clin. Exp. Allergy 26: 1150-1154), der p 7 (Shen et al. (1995) Clin. Exp. Allergy 25: 416-422), der f2 (Yuuki et al. (1997) Int. Arch. Allergy Immunol. 112: 44-48), der f3 (Nishiyama et al. (1995) FEBS Lett. 377: 62-66), der f7 (Shen et al. (1995) Clin. Exp. Allergy 25: 1000-1006); Mag 3 (Fujikawa et al. (1996) Mol. Immunol. 33: 311-319). Also of interest as allergens are the house dust mite allergens Tyr p2 (Eriksson et al. (1998) Eur. J. Biochem. 251: 443-447), Lep d1 (Schmidt et al. (1995) FEBS Lett. 370: 11-14), and glutathione S-transferase (O'Neill et al. (1995) Immunol Lett. 48: 103-107); the 25,589 Da, 219 amino acid polypeptide with homology with glutathione S-transferases (O'Neill et al. (1994) Biochim. Biophys. Acta. 1219: 521-528); Blo t 5 (Arruda et al. (1995) Int. Arch. Allergy Immunol. 107: 456-457); bee venom phospholipase A2 (Carballido et al. (1994) J. Allergy Clin. Immunol. 93: 758-767; Jutel et al. (1995) J. Immunol. 154: 4187-4194); bovine dermal/dander agents BDA 11 (Rautiainen et al. (1995) J. Invest. Dermatol. 105: 660-663) and BDA20 (Mantyjarvi et al. (1996) J. Allergy Clin. Immunol. 97: 1297-1303); the major horse allergen Equ c1 (Gregoire et al. (1996) J. Biol. Chem. 271: 32951-32959); Jumper ant M. pilosula allergen Myr p I and its homologous allergenic polypeptides Myr p2 (Donovan et al. (1996) Biochem. Mol. Biol. Int. 39: 877-885); 1-13, 14, 16 kD allergens of the mite Blomia tropicalis (Caraballo et al. (1996) J. Allergy Clin. Immunol. 98: 573-579); the cockroach allergens Bla g Bd90K (Helm et al. (1996) J. Allergy Clin. Immunol. 98: 172-80) and Bla g 2 (Arruda et al. (1995) J. Biol. Chem. 270: 19563-19568); the cockroach Cr-PI allergens (Wu et al. (1996) J. Biol. Chem. 271: 17937-17943); fire ant venom allergen, Sol i 2 (Schmidt et al. (1996) J. Allergy Clin. Immunol. 98: 82-88); the insect Chironomus thummi major allergen Chi t 1-9 (Kipp et al. (1996) Int. Arch. Allergy Immunol. 110: 348-353); dog allergen Can f 1 or cat allergen Fel d 1 (Ingram et al. (1995) J. Allergy Clin. Immunol. 96: 449-456); albumin, derived, for example, from horse, dog or cat (Goubran Botros et al. (1996) Immunology 88: 340-347); deer allergens with the molecular mass of 22 kD, 25 kD or 60 kD (Spitzauer et al. (1997) Clin. Exp. Allergy 27: 196-200); and the 20 kd major allergen of cow (Ylonen et al. (1994) J. Allergy Clin. Immunol. 93: 851-858).

Pollen and grass allergens include Hor v9 (Astwood and Hill (1996) Gene 182: 53-62, Lig v1 (Batanero et al. (1996) Clin. Exp. Allergy 26: 1401-1410); Lol p 1 (Muller et al. (1996) Int. Arch. Allergy Immunol. 109: 352-355), Lol p II (Tamborini et al. (1995) Mol. Immunol. 32: 505-513), Lol pVA, Lol pVB (Ong et al. (1995) Mol. Immunol. 32: 295-302), Lol p 9 (Blaher et al. (1996) J. Allergy Clin. Immunol. 98: 124-132); Par J I (Costa et al. (1994) FEBS Lett. 341: 182-186; Sallusto et al. (1996) J. Allergy Clin. Immunol. 97: 627-637), Par j 2.0101 (Duro et al. (1996) FEBS Lett. 399: 295-298); Bet v1 (Faber et al. (1996) J. Biol. Chem. 271: 19243-19250), Bet v2 (Rihs et al. (1994) Int. Arch. Allergy Immunol. 105: 190-194); Dac g3 (Guerin-Marchand et al. (1996) Mol. Immunol. 33: 797-806); Phl p 1 (Petersen et al. (1995) J. Allergy Clin. Immunol. 95: 987-994), Phl p 5 (Muller et al. (1996) Int. Arch. Allergy Immunol. 109: 352-355), Phl p 6 (Petersen et al. (1995) Int. Arch. Allergy Immunol. 108: 55-59); Cry j I (Sone et al. (1994) Biochem. Biophys. Res. Commun. 199: 619-625), Cry j II (Namba et al. (1994) FEBS Lett. 353: 124-128); Cor a 1 (Schenk etal. (1994) Eur. J. Biochem. 224: 717-722); cyn d1 (Smith et al. (1996) J. Allergy Clin. Immunol. 98: 331-343), cyn d7 (Suphiogluet al. (1997) FEBS Lett. 402: 167-172); Pha a 1 and isoforms of Pha a 5 (Suphioglu and Singh (1995) Clin. Exp. Allergy 25: 853-865); Cha o 1 (Suzuki et al. (1996) Mol. Immunol. 33: 451-460); profilin derived, e.g, from timothy grass or birch pollen (Valenta et al. (1994) Biochem. Biophys. Res. Commun. 199: 106-118); P0149 (Wu et al. (1996) Plant Mol. Biol. 32: 1037-1042); Ory s1 (Xu et al. (1995) Gene 164: 255-259); and Amb a V and Amb t 5 (Kim et al. (1996) Mol. Immunol. 33: 873-880; Zhu et al. (1995) J. Immunol. 155: 5064-5073).

Fungal allergens include, but are not limited to, the allergen Cla h III, of *Cladosporium herbarum* (Zhang et al. (1995) J. Immunol. 154: 710-717); the allergen Psi c 2, a fungal cyclophilin, from the basidiomycete *Psilocybe cubensis* (Homer et al. (1995) Int. Arch. Allergy Immunol. 107: 298-300); hsp 70 cloned from a cDNA library of *Cladosporium herbarum* (Zhang et al. (1996) Clin Exp Allergy 26: 88-95); the 68 kD allergen of *Penicillium notatum* (Shen et al. (1995) Clin. Exp. Allergy 26: 350-356); aldehyde dehydrogenase (ALDH) (Achatz et al. (1995) Mol Immunol. 32: 213-227); enolase (Achatz et al. (1995) Mol. Immunol. 32: 213-227); YCP4 (Id.); acidic ribosomal protein P2 (Id.).

Suitable food allergens include profilin (Rihs et al. (1994) Int. Arch. Allergy Immunol. 105: 190-194); rice allergenic cDNAs belonging to the alpha-amylase/trypsin inhibitor gene family (Alvarez et al. (1995) Biochim Biophys Acta 1251: 201-204); the main olive allergen, Ole e I (Lombardero et al. (1994) Clin Exp Allergy 24: 765-770); Sin a 1, the major allergen from mustard (Gonzalez De La Pena et al. (1996) Eur J Biochem. 237: 827-832); parvalbumin, the major allergen of salmon (Lindstrom et al. (1996) Scand. J Immunol. 44: 335-344); apple allergens, such as the major allergen Mal d 1 (Vanek-Krebitz et al. (1995) Biochem. Biophys. Res. Commun. 214: 538-551); and peanut allergens, such as Ara h I (Burks et al. (1995) J Clin. Invest. 96: 1715-1721).

This step constitutes the stimulation phase of the method described herein. Following exposure to the environmental allergen the activation of one or more genes associated with an allergic disorder is determined or measured. The rationale for this step is based upon the inventors' observation that particular genes are activated in atopic individuals relative to non-atopic individuals.
The step of analysing whether or not an allergy-associated gene is activated can be carried out using any standard techniques known in the art. For example, techniques such as reverse transcription polymerase chain reaction (RT-PCR) or DNA array analysis, ELISA, proteomic arrays, or intracellular staining as detected by flow cytometry may be used.

Many techniques for detecting gene expression can be employed. Technology systems for pharmacogenomic assays have recently been reviewed (Koch, WH: Nature Reviews Drug Discovery 2004 3 749-761). Gene expression may be measured in a biological sample directly, for example, by conventional Southern blotting to quantitate DNA, or Northern blotting to quantitate mRNA, using an appropriately labelled oligonucleotide hybridization probe, based on the known sequences of the allergy-associated nucleic acid molecules. Identification of mRNA from the allergy-associated nucleic acid molecules within a mixture of various mRNAs, is conveniently accomplished by the use of reverse transcriptase-polymerase chain reaction and an oligonucleotide hybridization probe which is labelled with a detectable moiety. Various labels, most commonly radioisotopes, particularly ³²P, may be employed. However, other techniques may also be employed, such as using biotin-modified nucleotides for introduction into a polynucleotide. The biotin then serves as the site for binding to avidin or antibodies, which may be labelled with a wide variety of labels, such as radioisotopes, fluorophores, chromophores, or the like. Keller, et al., DNA Probes, pp.149-213 (Stockton Press, 1989). Alternatively, antibodies may be employed that can recognise specific duplexes, including DNA duplexes, RNA duplexes, and DNA-RNA hybrid duplexes or DNA-protein duplexes. The antibodies in turn may be labelled and the assay may be carried out where the duplex is bound to a surface, so that upon the formation of duplex on the surface, the presence of antibody bound to the duplex can be detected.

In one method, mRNA in a biological sample is reverse transcribed to generate a cDNA strand. The cDNA may be amplified by conventional techniques, such as polymerase chain reaction, to provide sufficient amounts for analysis.

Amplification may also be used to determine whether a specific sequence is present, by using a primer that will specifically bind to the desired sequence, where the presence of an amplification product is indicative that a specific binding complex was formed. Alternatively, the mRNA sample is fractionated by electrophoresis, e.g. capillary or gel electrophoresis, transferred to a suitable support, e.g. nitrocellulose and then probed with a fragment of the transcription factor sequence. Other techniques may also find use, including oligonucleotide ligation assays, binding to solid-state arrays, etc. Detection of mRNA having the subject sequence is indicative gene expression of the transcription factor in the sample.

"Polymerase chain reaction," or "PCR," as used herein generally refers to a method for amplification of a desired nucleotide sequence in vitro, as described in U.S. Pat. No. 4,683,195. In general, the PCR method involves repeated cycles of primer extension synthesis, using two oligonucleotide primers capable of hybridizing preferentially to a template nucleic acid. Typically, the primers used in the PCR method will be complementary to nucleotide sequences within the template at both ends of or flanking the nucleotide sequence to be amplified, although primers complementary to the nucleotide sequence to be amplified also may be used. See Wang, et al., in PCR Protocols, pp.70-75 (Academic Press, 1990); Ochman, et al., in PCR Protocols, pp. 219-227; Triglia, et al., Nucl. Acids Res. 16:8186 (1988).

"Oligonucleotides" are short-length, single- or double-stranded polydeoxynucleotides that are chemically synthesized by known methods (involving, for example, triester, phosphoramidite, or phosphonate chemistry), such as described by Engels, et al., Agnew. Chem. Int. Ed. Engl. 28:716-734 (1989). They are then purified, for example, by polyacrylamide gel electrophoresis.
As used herein, the term "PCR reagents" refers to the chemicals, apart from the target nucleic acid sequence, needed to perform the PCR process. These chemicals generally consist of five classes of components: (i) an aqueous buffer, (ii) a water soluble magnesium salt, (iii) at least four deoxyribonucleotide triphosphates (dNTPs), (iv) oligonucleotide primers (normally two primers for each target sequence, the sequences defining the 5' ends of the two complementary strands of the double-stranded target sequence), and (v) a polynucleotide polymerase, preferably a DNA polymerase, more preferably a thermostable DNA polymerase, ie a DNA polymerase which can tolerate temperatures between 90□C and 100□C for a total time of at least 10 minutes without losing more than about half its activity.

The four conventional dNTPs are thymidine triphosphate (dTTP), deoxyadenosine triphosphate (dATP), deoxycitidine triphosphate (dCTP), and deoxyguanosine triphosphate (dGTP). These conventional triphosphates may be supplemented or replaced by dNTPs containing base analogues which Watson-Crick base pair like the conventional four bases, e.g. deoxyuridine triphosphate (dUTP).

A detectable label may be included in an amplification reaction. Suitable labels include fluorochromes, e.g. fluorescein isothiocyanate (FITC), rhodamine, Texas Red, phycoerythrin, allophycocyanin, 6-carboxyfluorexcein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; as well as others. The label may be a two stage system, where the amplified DNA is conjugated to biotin, haptens, or the like having a high affinity binding partner, e.g. avidin, specific antibodies, etc., where the binding partner is conjugated to a detectable label. The label may be conjugated to one or both of the primers. Alternatively, the pool of nucleotides used in the amplification is labelled, so as to incorporate the label into the amplification product.

Accordingly, in one preferred embodiment, once the allergy-associated gene mRNA has been reverse transcribed and amplified by PCR, it is detected by various means, including oligonucleotide probes. Oligonucleotide probes of the invention are DNA molecules that are sufficiently complementary to regions of contiguous nucleic acid residues within the allergy-associated gene nucleic acid to hybridise thereto, preferably under high stringency conditions. Exemplary probes include oligomers that are at least about 15 nucleic acid residues long and that are selected from any 15 or more contiguous residues of DNA of the present invention. Preferably oligomeric probes used in the present invention are at least about 20 nucleic acid residues long. The present invention also contemplates oligomeric probes which are 150 nucleic acid residues long or longer. Those of ordinary skill in the art realise that nucleic hybridisation conditions for achieving the hybridisation of a probe of a particular length to polynucleotides of the present invention can readily be determined. Such manipulations to achieve optimal hybridisation conditions for probes of varying lengths are well known in the art. See, e.g., Sambrook et al., Molecular Cloning: A Laboratory Manual, Second Edition, Cold Spring Harbor (1989*)*, incorporated herein by reference.

Preferably oligomeric probes of the present invention are labelled to render them readily detectable. Detectable labels may be any species or moiety that may be detected either visually or with the aid of an instrument. Commonly used detectable labels are radioactive labels such as, for example, ³²P, ¹⁴C, ¹²⁵I, ³H, and ³⁵S. Examples of fluorescer-quencher pairs may be selected from xanthene dyes, including fluoresceins, and rhodamine dyes. Many suitable forms of these compounds are widely available commercially with substituents on their phenyl moieties, which can be used as the site for bonding or as the bonding functionality for attachment to an oligonucleotide. Another group of fluorescent compounds is the naphthylamines which have an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9-isothiocyanatoacridine acridine orange; N-(p-(2-benzoaxazolyl)phenyl)maleimide; benzoxadiazoles, stilbenes, pyrenes, and the like. Most preferably, the fluorescent compounds are selected from the group consisting of VIC, carboxy fluorescein (FAM), Lightcycler^{®} 640 and Cy5.

Biotin-labelled nucleotides can be incorporated into DNA or RNA by such techniques as nick translation, chemical and enzymatic means, and the like. The biotinylated probes are detected after hybridisation, using indicating means such as avidin/streptavidin, fluorescent labelling agents, enzymes, colloidal gold conjugates, and the like. Nucleic acids may also be labelled with other fluorescent compounds, with immunodetectable fluorescent derivatives, with biotin analogues, and the like. Nucleic acids may also be labelled by means of attachment to a protein. Nucleic acids cross-linked to radioactive or fluorescent histone single-stranded binding protein may also be used. Those of ordinary skill in the art will recognise that there are other suitable methods for detecting oligomeric probes and other suitable detectable labels that are available for use in the practice of the present invention. Moreover, fluorescent residues can be incorporated into oligonucleotides during chemical synthesis.

Two DNA sequences are "substantially similar when at least about 85%, preferably at least about 90%, and most preferably at least about 95%, of the nucleotides match over the defined length of the DNA sequences. Sequences that are substantially similar can be identified for example in a Southern hybridisation experiment performed under stringent conditions as defined for that particular system. Defining appropriate hybridisation conditions is within the skill of the art. See e.g., Maniatis *et al*., DNA Cloning, vols. I and II. Nucleic Acid Hybridisation. However, briefly, "stringent conditions" for hybridisation or annealing of nucleic acid molecules are those that
(1) employ low ionic strength and high temperature for washing, for example, 0.015M NaCl/0.0015M sodium citrate/0.1% sodium dodecyl sulfate (SDS) at 50°C, or
(2) employ during hybridisation a denaturing agent such as formamide, for example, 50% (vol/vol) formamide with 0.1% bovine serum albumin/0.1% Ficoll/0.1% polyvinylpyrrolidone/50mM sodium phosphate buffer at pH 6.5 with 750mM NaCl, 75mM sodium citrate at 42°C. Another example is use of 50% formamide, 5 X SSC (0.75M NaCl, 0.075M sodium citrate), 50mM sodium phosphate (pH 6.8), 0.1% sodium pyrophosphate, 5 X Denhardt's solution, sonicated salmon sperm DNA (50µg/mL), 0.1% SDS, and 10% dextran sulfate at 42°C, with washes at 42°C in 0.2 X SSC and 0.1% SDS.

In a particularly preferred embodiment, the invention utilises a combined PCR and hybridisation probing system so as to exploit closed tube or homogenous assay systems, such as the use of FRET probes as disclosed in US patents Nos 6,140,054 and 6,174,670, the entirety of which are also incorporated herein by reference. In one of its simplest configurations, the FRET or "fluorescent resonance energy transfer" approach employs two oligonucleotides which bind to adjacent sites on the same strand of the nucleic acid being amplified. One oligonucleotide is labelled with a donor fluorophore which absorbs light at a first wavelength and emits light in response, and the second is labelled with an acceptor fluorophore which is capable of fluorescence in response to the emitted light of the first donor but not substantially by the light source exciting the first donor, and whose emission can be distinguished from that of the first fluorophore. In this configuration, the second or acceptor fluorophore shows a substantial increase in fluorescence when it is in close proximity to the first or donor fluorophore, as occurs when the two oligonucleotides come into close proximity when they hybridise to adjacent sites on the nucleic acid being amplified, for example in the annealing phase of PCR, forming a fluorogenic complex. As more of the nucleic acid being amplified accumulates, so more of the fluorogenic complex can be formed, and there is an increase in the fluorescence from the acceptor probe, which can be measured. Hence the method allows detection of the amount of product as it is being formed. In another simple embodiment, which is applicable to use of FRET probes in PCR based assays, one of the labelled oligonucleotides may also be a PCR primer used for PCR. In this configuration, the labelled PCR primer is part of the DNA strand to which the second labelled oligonucleotide hybridises, as described by Neoh et al (J Clin Path 1999;52:766-769.), von Ahsen et al (Clin Chem 2000;46:156-161), the entirety of which are incorporated herein by reference.

It will be appreciated by those of skill in the art that amplification and detection of amplification with hybridisation probes can be conducted in two separate phases, for example by carrying out PCR amplification first, and then adding hybridisation probes under conditions suitable to measure the amount of nucleic acid which has been amplified. However, a preferred embodiment of the present invention utilises a combined PCR and hybridisation probing system in order to employ the closed tube or homogenous assay systems, and is carried out on a Roche Lightcycler^{®} or other similarly specified or appropriately configured instrument.

Such systems would also be adaptable to the detection methods described here. Those skilled in the art will appreciate that such probes can be used for allele discrimination if appropriately designed for the detection of point-mutations, in addition to deletion and insertions. Alternatively or in addition, the unlabelled PCR primers may be designed for allele discrimination by methods well known to those skilled in the art (Ausubel 1989-1999).

It will also be appreciated by those skilled in the art that detection of amplification in homogenous and/or closed tubes can be carried out using numerous means in the art, for example using TaqMan® hybridisation probes in the PCR reaction and measurement of fluorescence specific for the target nucleic acids once sufficient amplification has taken place.

Those skilled in the art will be aware that other similar quantitative "real-time" and homogenous nucleic acid amplification/detection systems are available, such as those based on the TaqMan approach (US patents No. 5,538,848 and No. 5,691,146), fluorescence polarisation assays (eg. Gibson et al., Clin Chem, 1997; 43: 1336-1341), and the Invader assay (eg Agarwal P et al., Diagn Mol Pathol 2000 Sep; 9(3): 158-164; Ryan D et al, Mol Diagn 1999 Jun; 4(2): 135-144). Such systems would also be adaptable for use in the invention, enabling real-time monitoring of nucleic acid amplification.

In one embodiment of the invention an initial procedure involves the manufacture of the oligonucleotide matrices or microchips. The microchips contain a selection of immobilized synthetic oligomers, which are synthesized so as to contain complementary sequences for desired portions of transcription factor DNA. The oligomers are then hybridized with cloned or polymerase chain reaction (PCR) amplified transcription factor nucleic acids, said hybridization occurring under stringent conditions, outlined below. The high stringency conditions ensure that only perfect or near perfect matches between the sequence embedded in the microchip and the target sequence will occur during hybridization. After each initial hybridization, the chip is washed to remove most mismatched fragments. The reaction mixture is then denatured to remove the bound DNA fragments, which are subsequently labelled with a fluorescent marker. A second round of hybridization with the labelled DNA fragments is then carried out on sequence microchips containing a different set of immobilized oligonucleotides. These fragments may first be cleaved into smaller lengths. The different set of immobilized nucleotides may contain oligonucleotides needed for whole sequencing, partial sequencing, sequencing comparison, or sequence identification. Ultimately the fluorescence from this second hybridization step can be detected, for example using an epifluorescence microscope coupled to a CCD camera. (See US patent No. 5,851,772 incorporated herein by reference).

Gene expression may alternatively be measured by immunological methods, such as immunohistochemical staining of tissue sections and assay of cell culture or body fluids, to quantitate directly the expression of the gene product, cytokine transcription factor. With immunohistochemical staining techniques, a cell sample is prepared, typically by dehydration and fixation, followed by reaction with labelled antibodies specific for the gene product coupled, where the labels are usually visually detectable, such as enzymatic labels, fluorescent labels, luminescent labels, and the like. A particularly sensitive staining technique suitable for use in the present invention is described by Hsu, et al., Am. J. Clin. Path., 75:734-738 (1980). Antibodies useful for immunohistochemical staining and/or assay of sample fluids may be either monoclonal or polyclonal. The antibodies may conveniently be prepared against a synthetic peptide based on known DNA sequences of genes found herein to be allergy associated, such as cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 or CISH or comprising a sequence selected from the group consisting of sequences identified by probes 243610_at on human chromosome 9q21.13 at locus 138255, 1556097_at on human chromosome 15q25.2 and 242743_at on human chromosome 16p12.1 respectively.

For example, in appropriate cases the peptides may be used as an immunogens to generate anti-cytokine transcription factor antibodies. Such antibodies, which specifically bind to the products of the allergy-associated genes, are useful as standards in assays such as radioimmunoassay, enzyme-linked immunoassay, or competitive-type receptor binding assays or radioreceptor assays, as well as in affinity purification techniques.

Once the step of detecting the level of activation of one or more genes associated with an allergic disorder has been undertaken, a corresponding biological sample is contacted with a test agent. The term "agent" as used herein includes a compound, composition or matter, which might have the ability to modulate or change the presence or absence of activation of one or more genes associated with an allergic disorder that one wishes to test. In one embodiment, the agent is an siRNA molecule, an anti-sense oligonucleotide, a steroid, a β-2 agonist, a methylxanthine, a leukotriene modifier, an anti-cholinergic, a systemic corticosteroid, an antibody, an antagonist, an agonist or an anti-histamine.

The step of contacting the agent can be any method known in the art, but generally involves the incubation of the agent with the biological sample for sufficient time to elicit a response. After incubation, the step of detecting the presence or absence of activation of one or more genes associated with an allergic disorder is repeated. If any change in the activation is detected between the initial step and the step after incubation then the agent is considered to be useful in the prevention and/or treatment of an allergic disorder.

Generally, the terms "treating," "treatment" and the like are used herein to mean affecting an individual or animal, their tissue or cells to obtain a desired pharmacological and/or physiological effect. The effect may be prophylactic, i.e. it completely or partially prevents an allergic disorder or one or more signs or symptoms thereof, and/or may be therapeutic, i.e. effects a partial or complete cure of an allergic disorder. "Treating" as used herein includes any treatment of, or prevention of an allergic disorder in an animal, a mammal, particularly a human, and includes:
(a) preventing the allergic disorder from occurring in an animal which may be predisposed to the allergic disorder, but has not yet been diagnosed as having it;
(b) inhibiting the allergic disorder, i.e., arresting its development; or
(c) relieving or ameliorating the symptoms of the allergic disorder, i.e., causing a decrease in the symptoms of the allergic disorder.

In one embodiment, the treatment uses an antibody specifically reactive with a product of expression ( hereafter referred to as a "gene product" of a gene selected from DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, which is effective for decreasing a biological activity of the gene product. For example, by using immunogens derived from these gene products, peptides or polypeptides based on the cDNA sequences, antisera or monoclonal antibodies can be made using standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide or polypeptide, or an antigenic fragment or a fusion protein thereof which is capable of eliciting an antibody response. Techniques for conferring immunogenicity on a polypeptide or peptide include conjugation to carriers, or other techniques well known in the art. An immunogenic portion of the polypeptide or peptide can be administered in the presence of adjuvant. The progress of immunization can be monitored by detection of antibody titres in plasma or serum. Standard ELISA or other immunoassays can be used with the immunogen as agent to assess the levels of antibodies. In a preferred embodiment, the antibodies are immunospecific for antigenic determinants of the products of the allergy-associated genes disclosed herein.

Following immunization of an animal with the antigenic preparation antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4:72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with the gene products of the present invention and monoclonal antibodies isolated from a culture comprising such hybridoma cells.

The term "antibody" as used herein is intended to include fragments thereof which are also specifically reactive with one of the subject gene products. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific, single-chain, and chimeric and humanized molecules having affinity for the gene products, conferred by at least one CDR region of the antibody.

In certain preferred embodiments, an antibody of the invention is a monoclonal antibody, and in certain embodiments the invention makes available methods for generating novel antibodies. For example, a method for generating a monoclonal antibody that binds specifically to a gene product of the invention may comprise administering to a mouse an amount of an immunogenic composition comprising the gene, effective to stimulate a detectable immune response, obtaining antibody-producing cells (e.g. cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to the gene. Once obtained, a hybridoma can be propagated in a cell culture, optionally in culture conditions where the hybridoma-derived cells produce the monoclonal antibody that binds specifically to the gene product. The monoclonal antibody may be purified from the cell culture.

Another aspect of the invention relates to the use of "antisense" therapy. As used herein, antisense therapy refers to administration or in situ generation of oligonucleotide probes or their derivatives which specifically hybridize (e.g. binds) under cellular conditions with the cellular mRNA and/or genomic DNA encoding one of the subject allergy associated genes so as to inhibit expression of that molecule, e.g. by inhibiting transcription and/or translation. The binding may be by conventional base pair complementarity, or, for example, in the case of binding to DNA duplexes, through specific interactions in the major groove of the double helix. In general, antisense therapy refers to the range of techniques generally employed in the art, and includes any therapy, which relies on specific binding to oligonucleotide sequences.

An antisense construct of the present invention can be delivered, for example, as an expression plasmid which, when transcribed in the cell, produces RNA which is complementary to at least a unique portion of the cellular mRNA which encodes DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 or CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively. Alternatively, the antisense construct is an oligonucleotide probe, which is generated *ex vivo* and which, when introduced into the cell causes inhibition of expression by hybridizing with the mRNA and/or genomic sequences encoding DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 or CISH or which comprise a sequence selected from the group consisting of sequences identified by probes 243610_at on human chromosome 9q21.13 at locus 138255, 1556097_at on human chromosome 15q25.2 and 242743_at on human chromosome 16p12.1 respectively. Such oligonucleotide probes are preferably modified oligonucleotide which are resistant to endogenous nucleases, e.g. exonucleases and/or endonucleases, and is therefore stable *in vivo*. Exemplary nucleic acid molecules for use as anti-sense oligonucleotides are phosphoramidate, phosphothioate and methylphosphonate analogues of DNA (see also U.S. Pat. Nos. 5,176,996; 5,264,564; and 5,256,775). Additionally, general approaches to constructing oligomers useful in antisense therapy have been reviewed, for example, by van der Krol et al., (1988) Biotechniques 6:958-976; and Stein et al., (1988) Cancer Res 48:2659-2668.

In another embodiment, the treatment of the allergic disorder uses RNA interference (RNAi). RNAi is a form of sequence-specific, post-transcriptional gene silencing in animals, elicited by double-stranded RNA (dsRNA) that is homologous in sequence to the silenced gene. Elbashir et al., Nature, 2001, 411, 494-498. dsRNA triggers the specific degradation of homologous RNAs, only within the region of homology. The dsRNA is processed to 21- to 23-nucleotide fragments, sometimes called short interfering RNAs (siRNAs) which are believed to be the guide fragments for sequence-specific mRNA degradation. The processing of longer dsRNA to these short siRNA fragments is believed to be accomplished by RNase III. Elbashir *et al*., *ibid*., Elbashir, et al., Genes and Devel., 2001, 15, 188-200.

Therefore, once an individual afflicted with an allergic disorder has been diagnosed and a potentially useful agent, or combination of agents, has been identified by the methods disclosed herein, then an "effective amount" of the agent(s) is administered to the animal.
The terms "administration," administering," and "administered" are used herein interchangeably. The agent may be administered orally including sublingual, topically, or parenterally in dosage unit formulations containing conventional non-toxic pharmaceutically acceptable carriers, adjuvants, and vehicles.

In clinical settings, the therapeutic or prophylactic agent of the present invention can be introduced into an animal by any of a number of methods, each of which is familiar in the art. For instance, a pharmaceutical preparation of the gene delivery system or peptide can be introduced systemically, e.g. by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof. In other embodiments, initial delivery of the antisense or siRNA is more limited with introduction into the animal being quite localized. For example, the gene delivery vehicle or peptide can be introduced by catheter (see U.S. Pat. No. 5,328,470) or by stereotactic injection (e.g. Chen et al. (1994) PNAS 91:3054-3057).

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index, which can be expressed as the ratio LD50/ED50. Compounds which exhibit large therapeutic indices are preferred. While compounds that exhibit toxic side effects may be used, care should be taken to design a delivery system which targets such compounds to the site of affected tissue in order to minimize potential damage to uninfected cells and thereby reduce side effects.

The data obtained from cell culture assays and animal studies can be used in formulating a range of dosage for use in humans. The dosage of such compounds preferably lies within a range of circulating concentrations which include the ED50, with little or no toxicity. The dosage may vary within this range, depending upon the dosage form employed and the route of administration utilized. For any compound used in the method of the invention, the therapeutically effective dose can be initially estimated from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The invention will now be further described by way of reference only to the following non-limiting examples. It should be understood, however, that the examples following are illustrative only, and should not be taken in any way as a restriction on the generality of the invention described above. In particular, while the invention is described in detail in relation to the detection of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH mRNA from HDM-exposed PMBC, it will be clearly understood that the findings herein are not limited to these specific allergens or methods.

### EXAMPLE 1 USE OF MICROARRAY TO DETERMINE SPECIFIC EXPRESSION OF mRNA IN ALLERGIC AND NON-ALLERGIC SUBJECTS IN RESPONSE TO ALLERGEN

Blood samples were obtained from 10 allergic individuals, who were selected on the basis of positive serum IgE responses to House Dust Mite (HDM), together with samples from 10 non-allergic controls who were tested for the presence of HDM-specific IgE in serum and were all negative. All 10 allergic subjects showed a wheal size of 5-14 mm in response to a skin prick test with allergen, whereas all non-allergic subjects showed a negative response. The presence of IgE directed against HDM was detected by the radioallergosorbent immunoassay capture test system, using the RAST (CAP) (Pharmacia, Australia), and the allergic volunteers in this study displayed RAST (CAP) scores ≥2.

Freshly-isolated peripheral blood mononuclear cells (PBMC) were resuspended at 1 x 10⁶ cells/ml, and 0.5ml of the cell suspension was cultured for 16, 24 or 48 hours at 37°C, 5% CO₂ in round bottom plates in serum-free medium AIM_V⁴ (Life Technologies, Mulgrave, Australia) supplemented with 4 x 10⁻⁵ 2-mercaptoethanol, with or without the addition of 30µg/mlof whole extract of HDM (*Dermatophagoides pteronyssinus*, CSL Limited, Parkville, Australia).

At each time point, equal-sized aliquots of cells were centrifuged and the cell pellets were used immediately for total RNA extraction. Alternatively, Dynabeads™ were used for positive selection of CD8⁺ T cells followed by positive selection of CD4⁺ T cells, prior to extraction of total RNA. Extraction of the RNA was performed by standard techniques. Total RNA was extracted using TRIZOL (Invitrogen) followed by a Rneasy minikit (QIAGEN). Alternatively a Totally RNA extraction kit (Ambion, Austin, Texas, USA) could be used. If desired, messenger RNA can then be purified from 2mg of total RNA using a MessageMaker™ kit (Invitrogen, The Netherlands).

The extracted RNA from the 10 individuals in each group (allergic and non-allergic) was pooled, and then labelled and hybridised to Affymetrix™ Genechip^{R} U133A or U133plus2 arrays, using the standard Affymetrix™ protocols. Full details of the arrays and protocols are available on the Affymetrix website (http://www.affymetrix.com/index.affx). The U133A arrays provides probe sets corresponding to over 39,000 human genomes, while the U133plus2 array provides probe sets corresponding to over 47,000 transcripts, including all those from the U133A arrays. All of the corresponding nucleic acid sequences are available in publicly-available databases. Samples of the individual RNAs in the pools were kept separate for subsequent Taqman^{™} PCR validation studies (see Example 6 below).

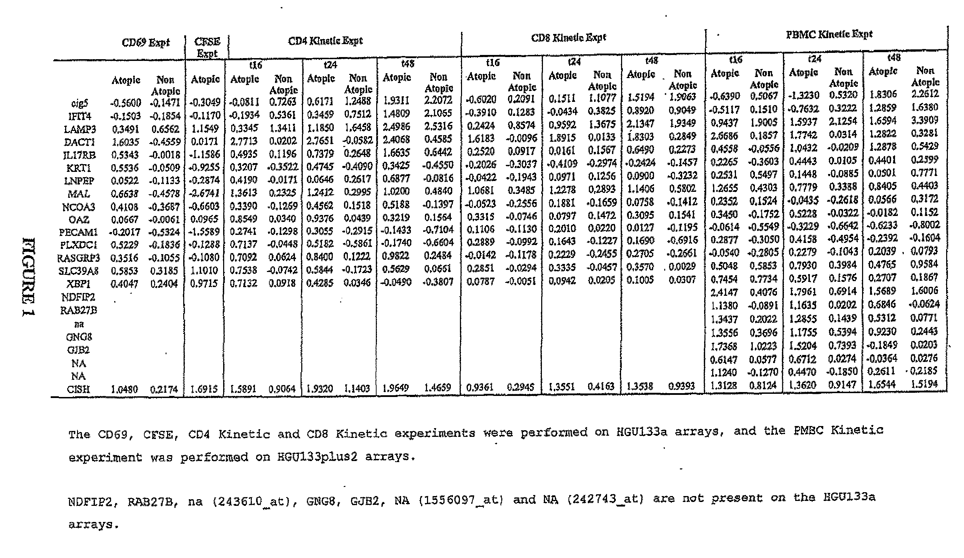

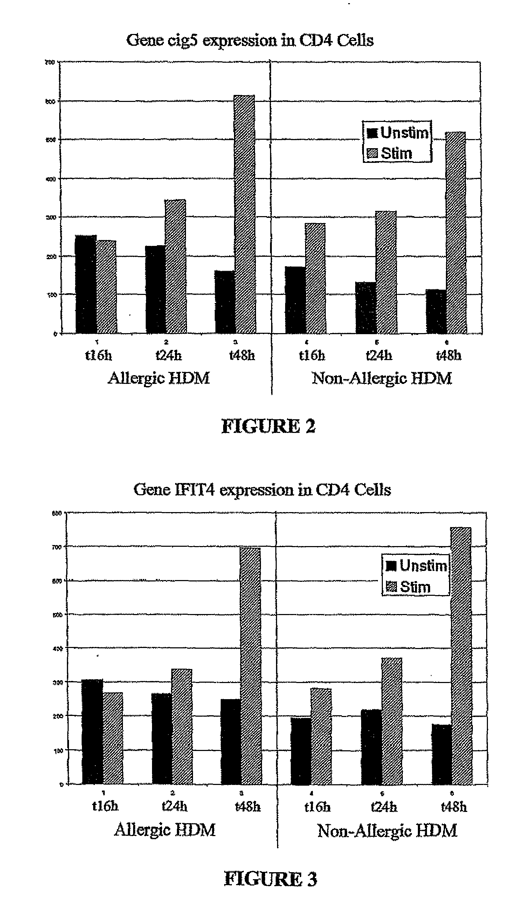

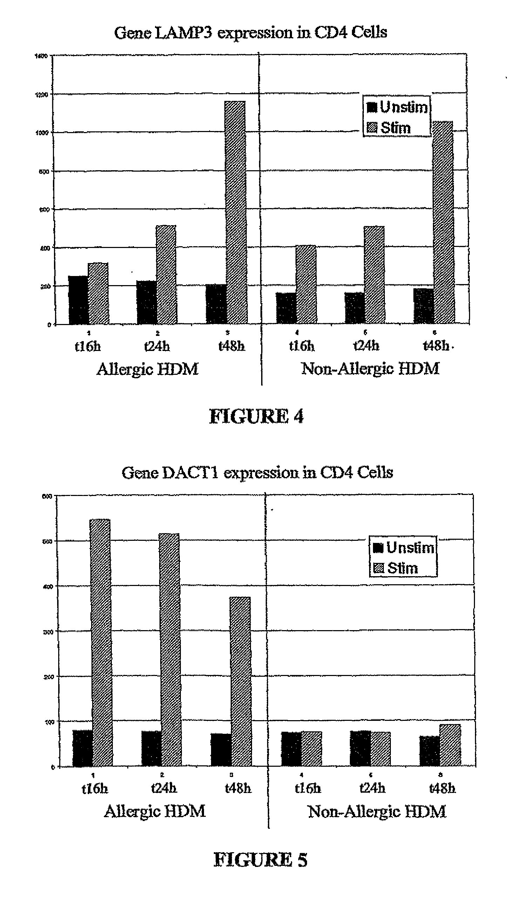

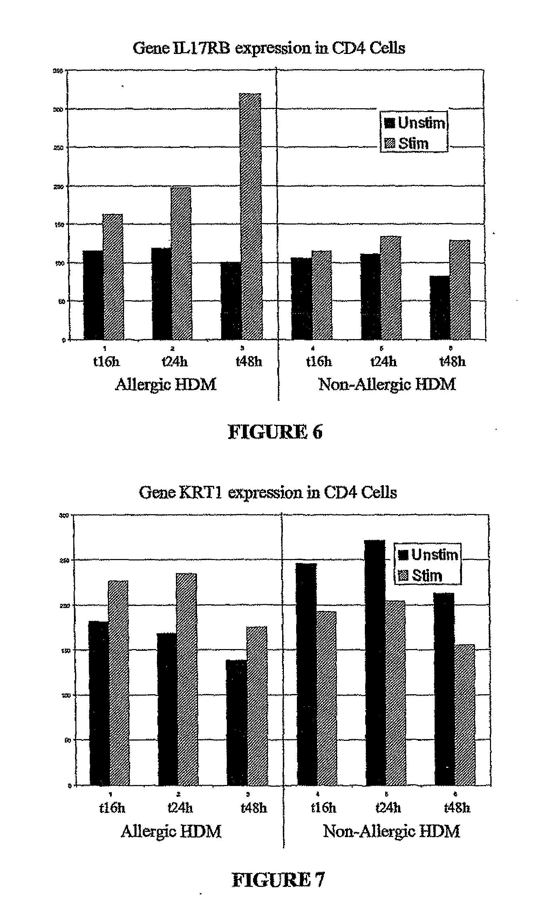

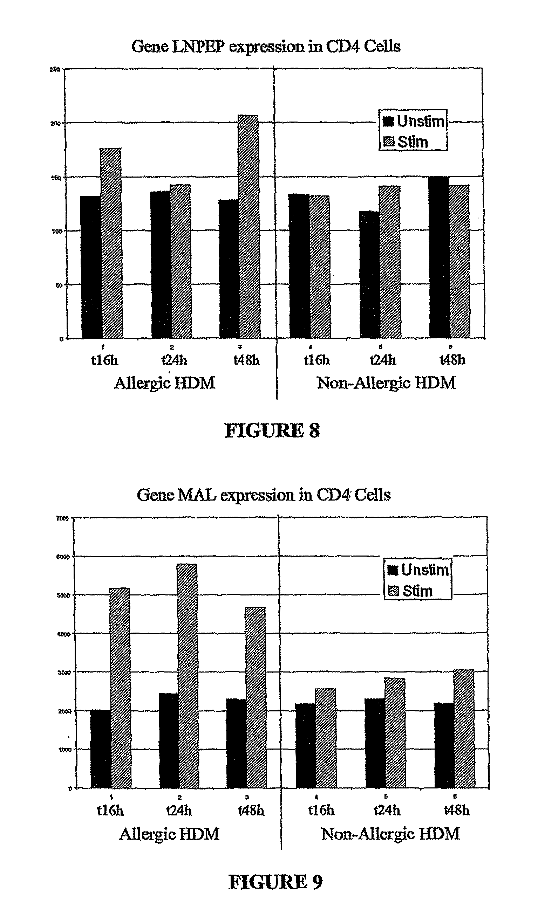

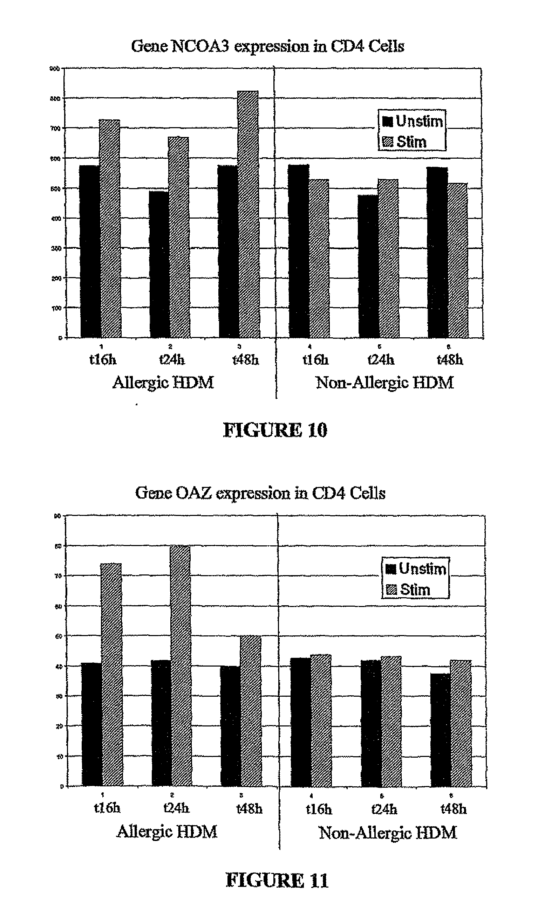

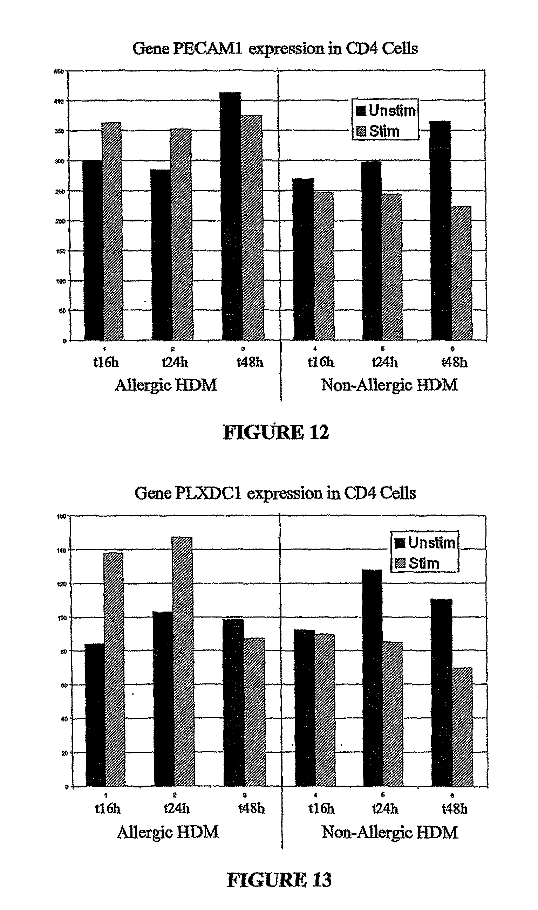

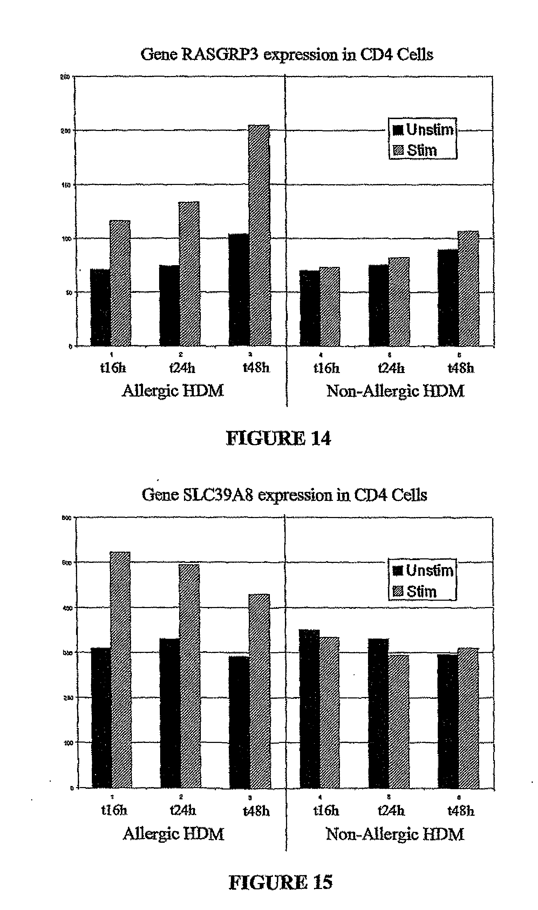

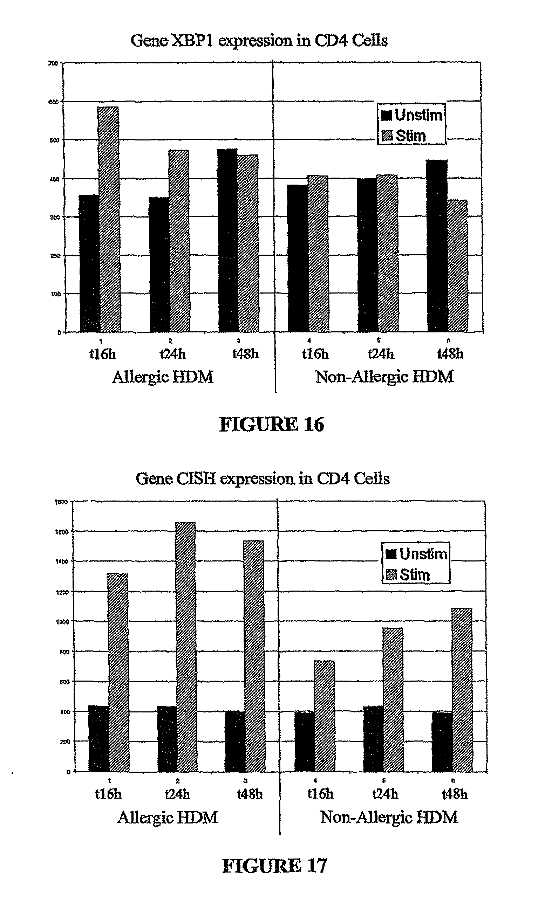

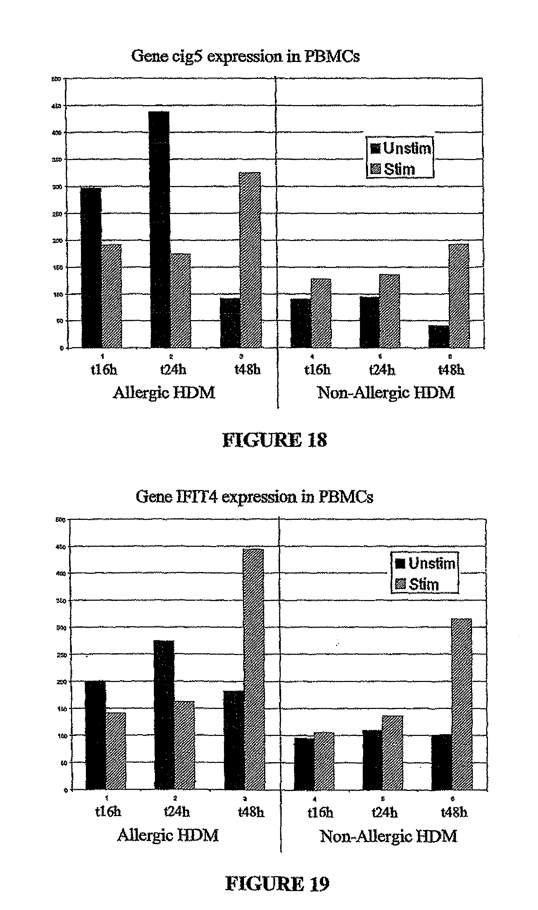

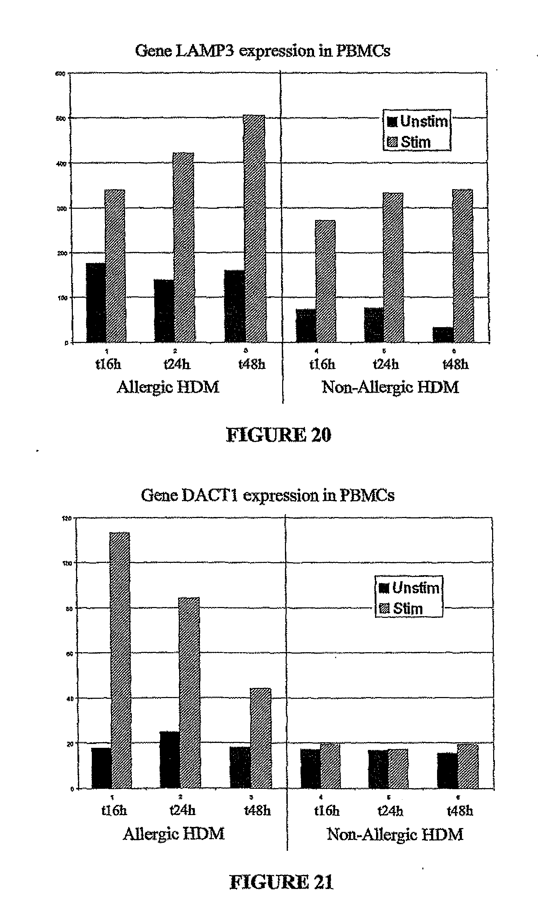

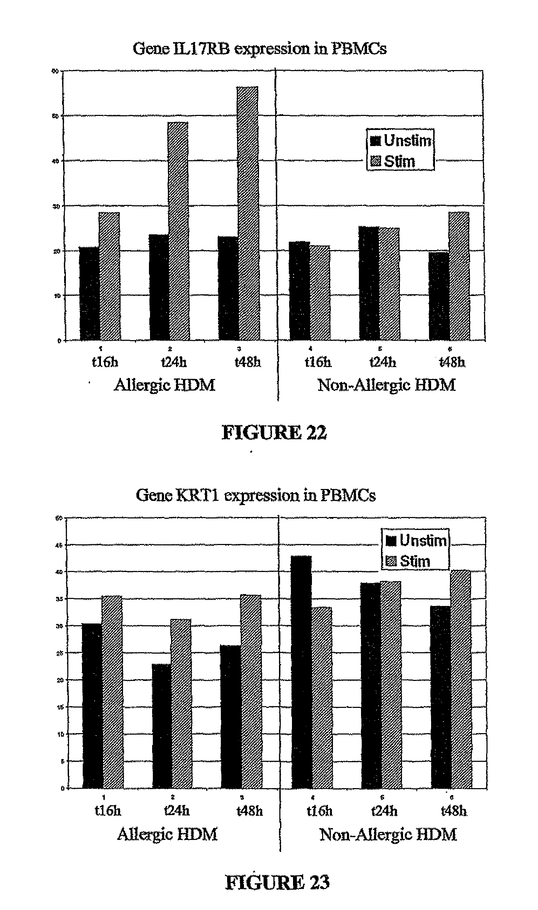

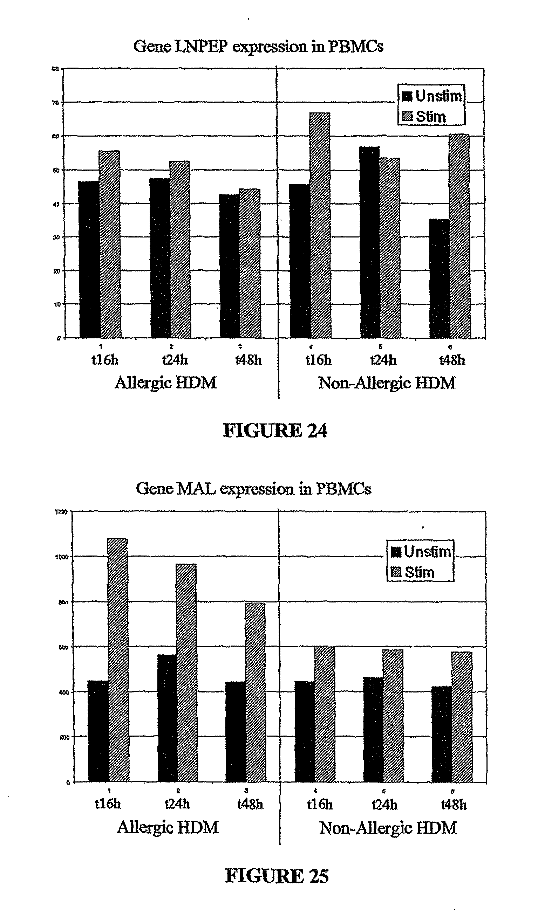

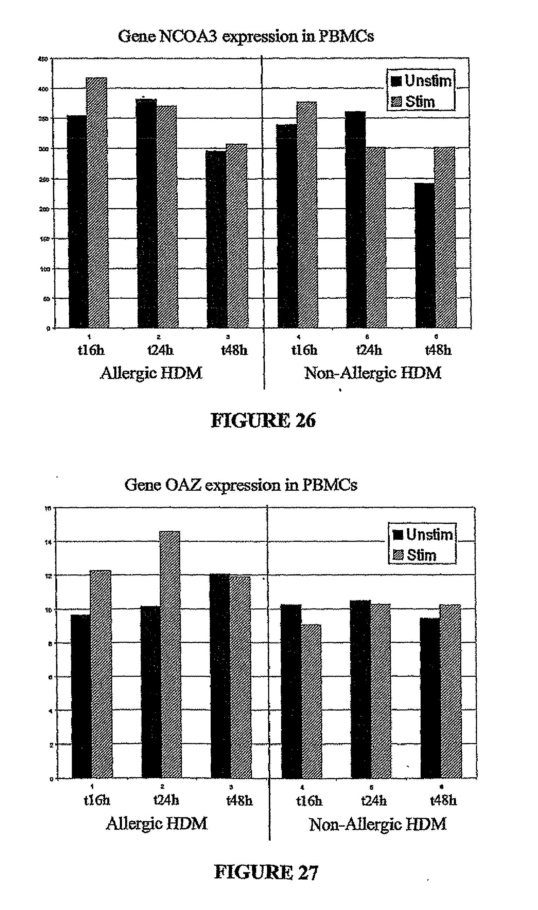

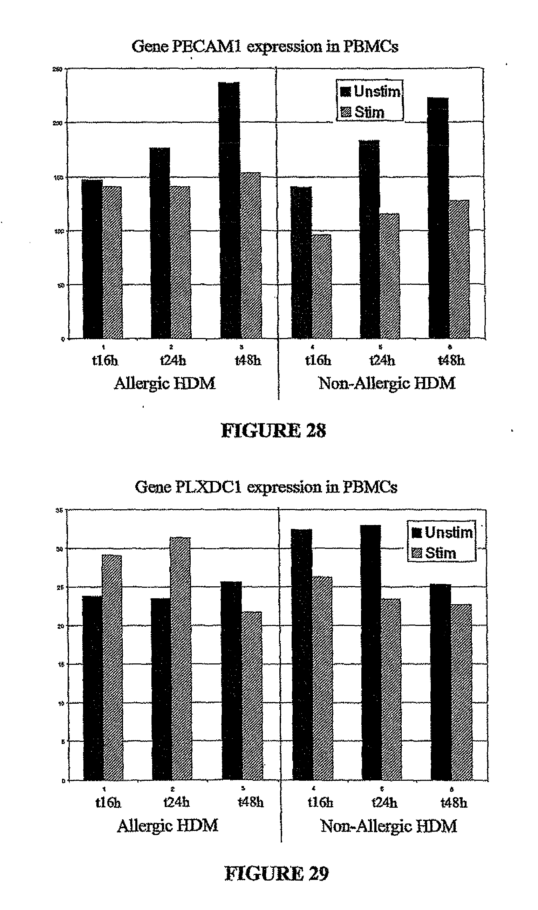

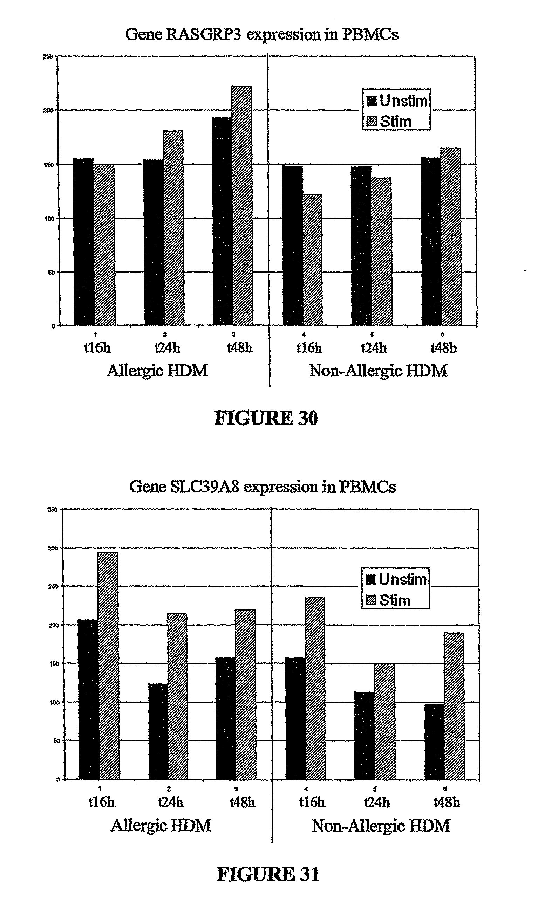

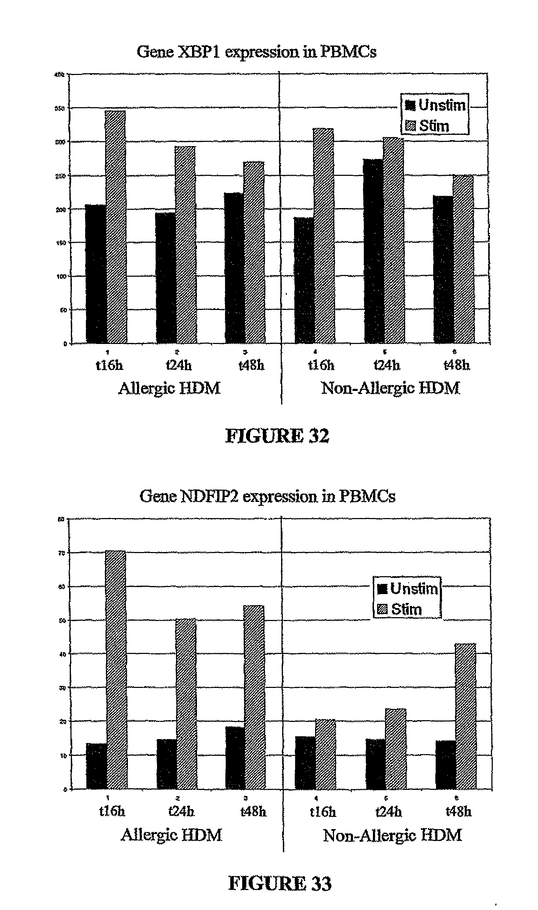

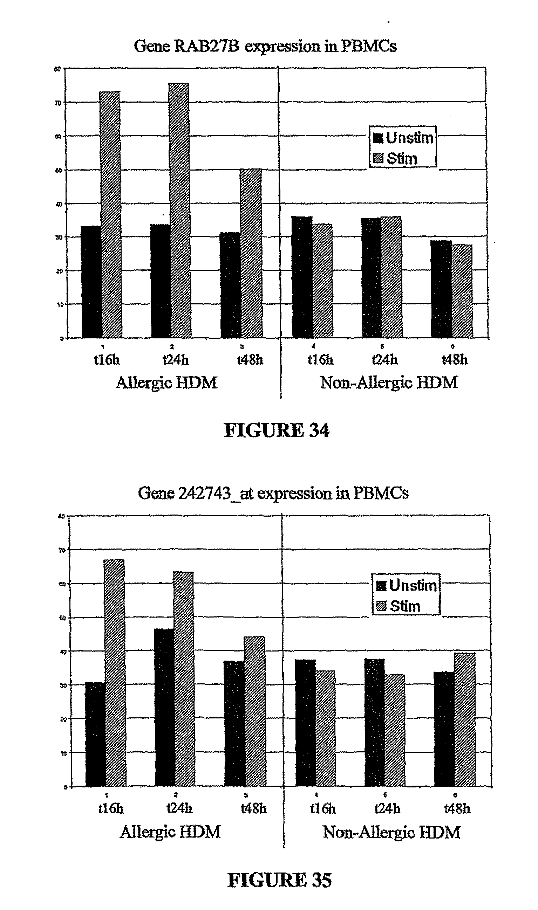

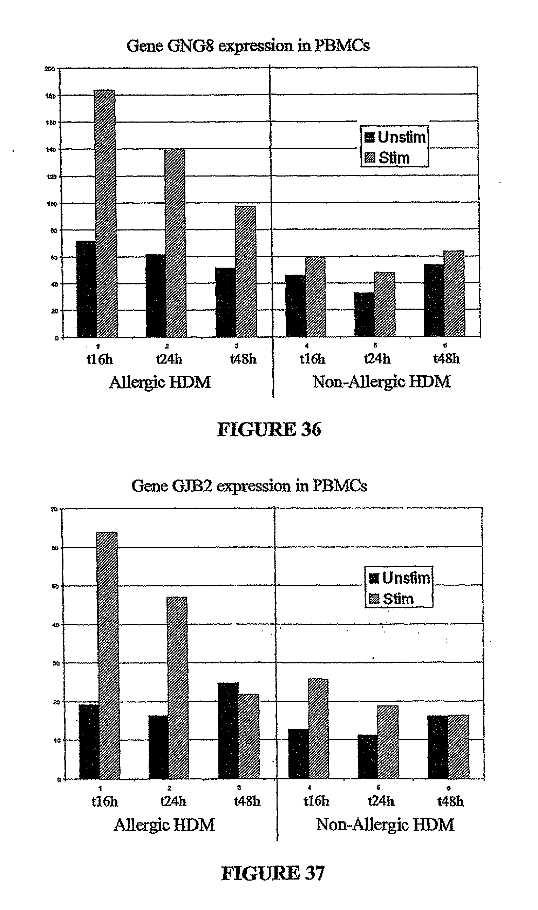

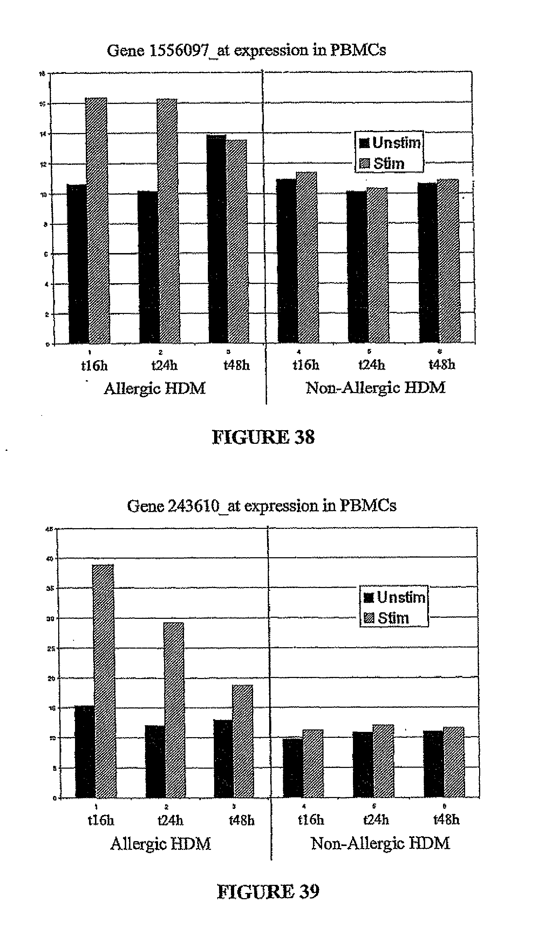

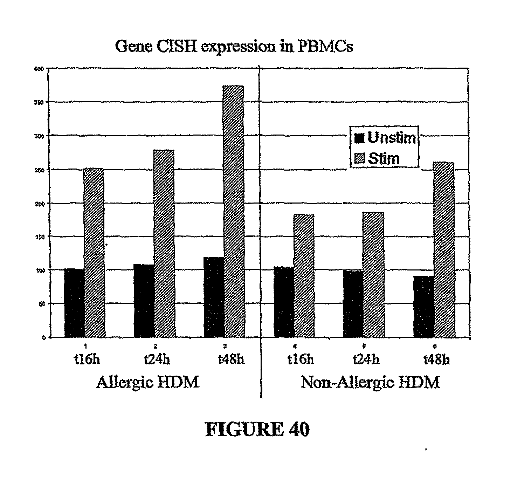

Data from these microarray experiments, and those of Examples 2-3 below, are shown in Figure 1 as fold expression (stimulated vs unstimulated cultures) on a log2 scale. Data were analysed with the rma algorithm using the statistical package R (Irizarry R.A. et al. 2003, Biostatistics 4(2):249-64). Genes were considered to be differentially expressed in stimulated and unstimulated cultures if the fold-change value was greater than the cut-off value (background noise), which is shown for each experiment. Cut-off values were determined on the basis of the standard deviation of the noise for each experiment. Genes which were consistently expressed at different levels in samples from allergic and non-allergic individuals, i.e. genes with large fold-change values between allergic individuals and non-allergic individuals were then identified. A total of 23 genes showed differential expression; of these 16 were identified using the U133a arrays, and a further 7 were identified using the U133plus2 arrays. Illustrative expression patterns of the selected genes are shown in Figures 2 to 40. Data in these figures are shown as absolute expression intensity levels on a linear scale.

The microarray data summarized in Figures 2 to 40 show that cig5, IFIT4 and LAMP3 are upregulated in non-allergic individuals, i.e. these genes are upregulated in HDM-stimulated cultures compared to unstimulated cultures to a greater extent in the non-allergic individuals than the allergic individuals, at least at 16 and 24 hours of culture. The remaining 20 genes are upregulated in the allergic individuals, and indeed KRT1, PECAM1 and PLXDC1 are actually down regulated in the non-allergic individuals.

These data were interpreted as follows: If the genes are "protective" i.e. if the expression of these genes is associated with absence of allergy, then allergic individuals will have expression levels which are lower than those non-allergic individuals. For example, in the PBMC kinetic experiment at 48 hours post-stimulation cig5 shows a figure of 1.8306 for allergic individuals, which is lower than the corresponding figure of 2.2612 for non-allergic individuals. Similarly, IFIT4 shows a figure of 1.2859 for allergic individuals, which is lower than the corresponding figure of 1.6380 for non-allergic individuals. The expression of these genes is therefore considered to be "protective".

Genes that are indicative of allergic disorder are those in which the expression level for allergic individuals is higher than that for non-allergic individuals. For example, DACT1 in the PBMC kinetic experiment at 48 days post-stimulation shows a figure of 1.2822 for allergic individuals, which is higher than the corresponding figure for non-allergic individuals at 0.3281. Similarly, IL17RB shows a figure of 1.2878 for allergic individuals, which is higher than the corresponding figure for non-allergic individuals at 0.5429. The expression of these genes is therefore considered to be "predictive of a predisposition for allergy".

### EXAMPLE 2 USE OF MICROARRAY ANALYSIS TO DETERMINE SPECIFIC EXPRESSION OF mRNA ISOLATED FROM CD69⁺ CELLS FROM ALLERGIC AND NON-ALLERGIC SUBJECTS

Blood samples were obtained from 4 allergic individuals, who were selected on the basis of positive serum IgE responses to House Dust Mite (HDM), and from 4 non-allergic controls that were tested for the presence of HDM-specific IgE in serum and were all negative. The presence of IgE directed against HDM was detected by the RAST (CAP) system (Pharmacia, Australia), and the allergic volunteers in this study displayed RAST (CAP) scores ≥2.

PBMCs from all individuals were cultured in the presence or absence of HDM (10µg/ml) for 14 hours, as described in Example 1. Following the 14 hour stimulation, monocytes and B cells, which express high levels of CD69, were removed using Dynabeads™ coated with CD14 and CD19 in accordance with the manufacturer's instructions. Activated CD69⁺ T cells were then positively selected from the remaining cell population, using Dynabeads™ coated with anti-CD69 monoclonal antibody. RNA was extracted, labelled and hybridised to Affymetrix™ U133a arrays using the standard Affymetrix™ protocols, as described in Example 1.

The results of these experiments are shown in Figure 1 column 1, and the data analysis was performed as described in Example 1. Again it can be seen that for those genes which are considered "protective", allergic individuals had lower levels of expression relative to non-allergic individuals, while those genes considered to be predictive of a predisposition to develop allergy were more highly expressed in allergic individuals than in non-allergic individuals. It is worthy of note that the closer the figures are to zero, the greater the level of interference from the background and therefore the less accurate the test.

### EXAMPLE 3 USE OF MICROARRAY ANALYSIS TO DETERMINE SPECIFIC EXPRESSION OF mRNA ISOLATED FROM RECENTLY-DIVIDED CELLS FROM ALLERGIC AND NON-ALLERGIC SUBJECTS

PBMCs from 4 allergic and 4 non-allergic individuals were labelled with 5µm carboxy-fluorescein diacetate, succinimidyl ester (CFSE) by standard procedures, then stimulated with HDM (10µg/ml) for 6 days as described in Example 1. The CFSE fluorescence stain is used to monitor cell division. Cells which are the progeny of recent cell division events show a low degree of staining (CFSE^{low}); non-dividing cells are strongly stained. Live progeny cells (CFSE^{low}) were sorted by flow cytometry, rested overnight, and then stimulated with PMA and ionomycin for 6 hours. RNA was extracted, labelled and hybridised to Affymetrix™ U133a arrays using the standard Affymetrix™ protocols as described above.

The results of these experiments are shown in Figure 1 column 2, and the data analysis was performed as described in Example 1. In these experiments there was very poor proliferation of HDM-specific cells from non-allergic subjects, and so sufficient RNA to analyse only the allergic subjects was obtained. However, this experiment does demonstrate that it is possible to concentrate specific T-cells by initial generation of a "cell line" which is then re-stimulated using agents known in the art to provide "optimal" stimulation e.g. PMA/ionomycin. This is in contrast to the more subtle stimulus of specific allergen at low concentration applied to unfractionated T-cells using total PBMC.

### EXAMPLE 4 VALIDATION OF RESULTS FROM EXAMPLE 1

IL-4 is the essential growth factor for all Th2 cells.
Therefore to confirm the "Th2 status" of each PBMC sample, real-time quantitative PCR was performed in order to measure expression levels of the index gene IL-4 in RNA extracts from 48hr cell pellets from the individual samples used to generate the pools for the kinetic experiment described in Example 1, using ABI Prism 7900HT Sequence Detection System.

Standard PCR premixes were prepared using QuantiTect SYBRGreen PCR Master Mix (QIAGEN), containing 2.5mM MgCl₂ (final concentration). SYBRGreen binds to all double-stranded DNA, so no probe is needed. Primers were used at a final concentration of 0.3µM. Standard conditions were used, except that 15 minutes instead of 10 minutes was used for HotStar Taq polymerase activation. In addition, a dissociation step was included and melt curve analysis performed to confirm amplification of a single product. Amplified products have been or will be sequenced to confirm specific amplification of the target of interest.

The primers used for the PCR were:
IL-4 Forward: 5'AACAGCCTCACAGAGCAGAAGACT3' (SEQ ID NO:47)
   IL-4 Reverse: 5'CAGCGAGTGTCCTTCTCATGGT3' (SEQ ID NO:48)
LAMP3 forward: 5'GCGTCCCTGGGCGTAATTT3' (SEQ ID NO:5)
   LAMP3 reverse: 5'TGGTTGCTTAGCTGGTTGCT3' (SEQ ID NO:6)
DACT1 forward: 5'AACTCGGTGTTCAGTGAGTGT3' (SEQ ID NO:7)
   DACT1 reverse: 5'GGAGAGGGAACGGCAAACT3' (SEQ ID NO:8)
PLXDC1 forward: 5'CCTGGGCATGTGTCAGAGC3' (SEQ ID NO:23)
   PLXDC1 reverse: 5'GGTGTTGGAGAGTATTGTGTGG3' (SEQ ID NO:24)
cig5 forward: 5'CAAGACCGGGGAGAATACCTG3' (SEQ ID NO:1)
   cig5 reverse: 5'GCGAGAATGTCCAAATACTCACC3' (SEQ ID NO:2)
IFIT4 forward: 5'GAGTGAGGTCACCAAGAATTC3' (SEQ ID NO:3)
   IFIT4 reverse: 5'CACTCTATCTTCTAGATCCCTTGAGA3' (SEQ ID NO:4)
MAL forward: 5'TCGTGGGTGCTGTGTTTACTCT3' (SEQ ID NO:15)
   MAL reverse: 5' CAGTTGGAGGTTAGACACAGCAA3' (SEQ ID NO:16)
NCOA3 forward: 5'CCTGTCTCAGCCACGAGCTA3' (SEQ ID NO:17)
   NCOA3 reverse: 5'TCCTGAAAGATCATGTCTGGTAA3' (SEQ ID NO:18)
PECAM1 forward: 5'AGTCCAGATAGTCGTATGTGAAATGC3' (SEQ ID NO:21)
   PECAM1 reverse: GGTCTGTCCTTTTATGACCTCAAAC3' (SEQ ID NO:22)
SLC39A8 forward: 5'GCAGTCTTACAGCAATTGAACTTT3' (SEQ ID NO:27)
   SLC39A8 reverse: 5'CCATATCCCCAAACTTCTGAA3' (SEQ ID NO:28)
XBP1 forward: 5'GTAGATTTAGAAGAAGAGAACCAAAAAC3' (SEQ ID NO:29)
   XBP1 reverse: 5'CCCAAGCGCTGTCTTAACTC3' (SEQ ID NO:30)
NDFIP2 forward: 5'AGTGGGGAATGATGGCATTTT3' (SEQ ID NO:31)
NDFIP2 reverse: AAATCCGCAGATAGCACCA3' (SEQ ID NO:32)
RAB27B forward: 5'CAGAAACTGGATGAGCCAACT3' (SEQ ID NO:33)
   RAB27B reverse: 5'GACTTCCCTCTGATCTGGTAGG3' (SEQ ID NO:34)
243610_at forward: 5'TGCATTGACAACGTACTCAGAA3' (SEQ ID NO:35)
   243610_at reverse: 5'TCATCTTGACAGGGATAAGCAT3' (SEQ ID NO:36)
GNG8 forward: 5'GAACATCGACCGCATGAAGGT3' (SEQ ID NO:37)
   GNG8 reverse: 5'AGAACACAAAAGAGGCGCTTG3' (SEQ ID NO:38)
GJB2 forward: 5'GCTTCCTCCCGACGCAGA3' (SEQ ID NO:39)
   GJB2 reverse: 5'AACGAGGATCATAATGCGAAA3' (SEQ ID NO:40)
1556097_at forward: 5'TCTTATTTCACTTTCTCAACTCATCA3' (SEQ ID NO:41)
   1556097_at reverse: 5'GGCATAACCTGAATGTATAATTCAA3' (SEQ ID NO:42)
242743_at forward: 5'GAAAAAGCTGTTGAGTGAAGAAGACT3' (SEQ ID NO:43)
   242743_at reverse: 5'TGCAGGATGAGCAATGCTGAGA3' (SEQ ID NO:44)
   and
CISH forward: 5'GGGAATCTGGCTGGTATTGG3' (SEQ ID NO:45)
   CISH reverse: 5'TTCTGGCATCTTCTGCAGGTGTT3' (SEQ ID NO:46).

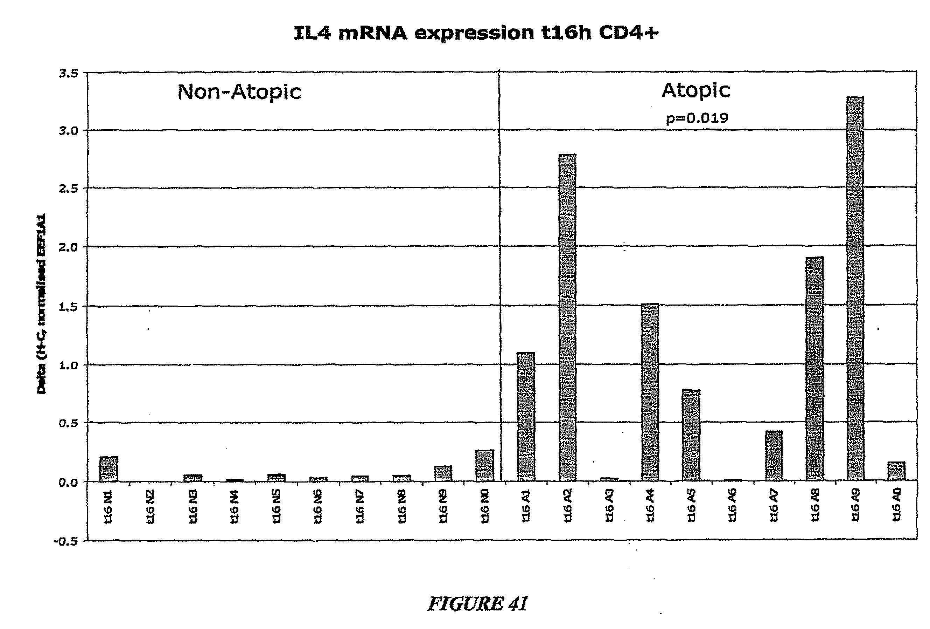

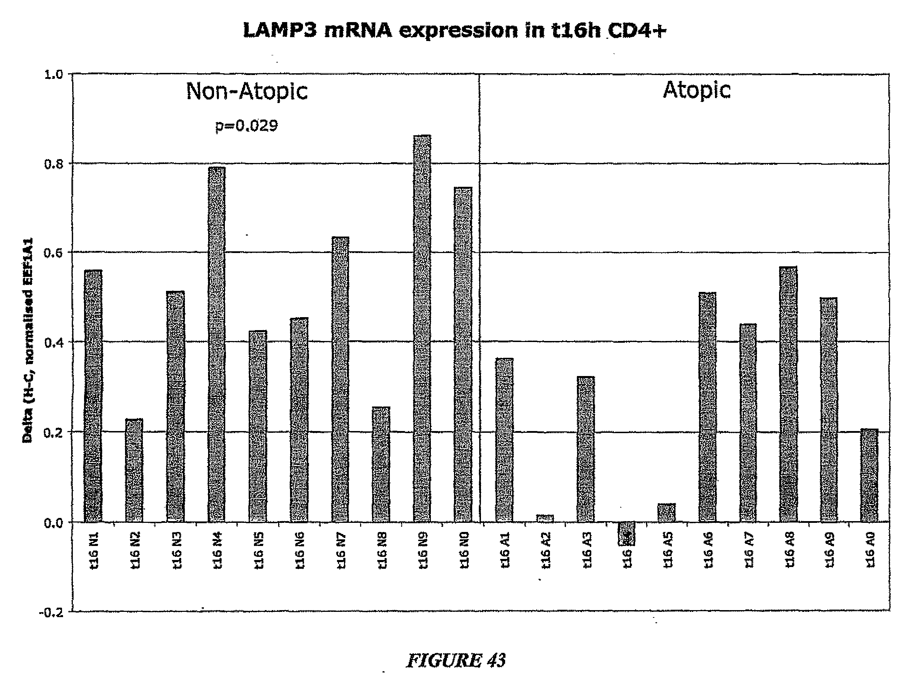

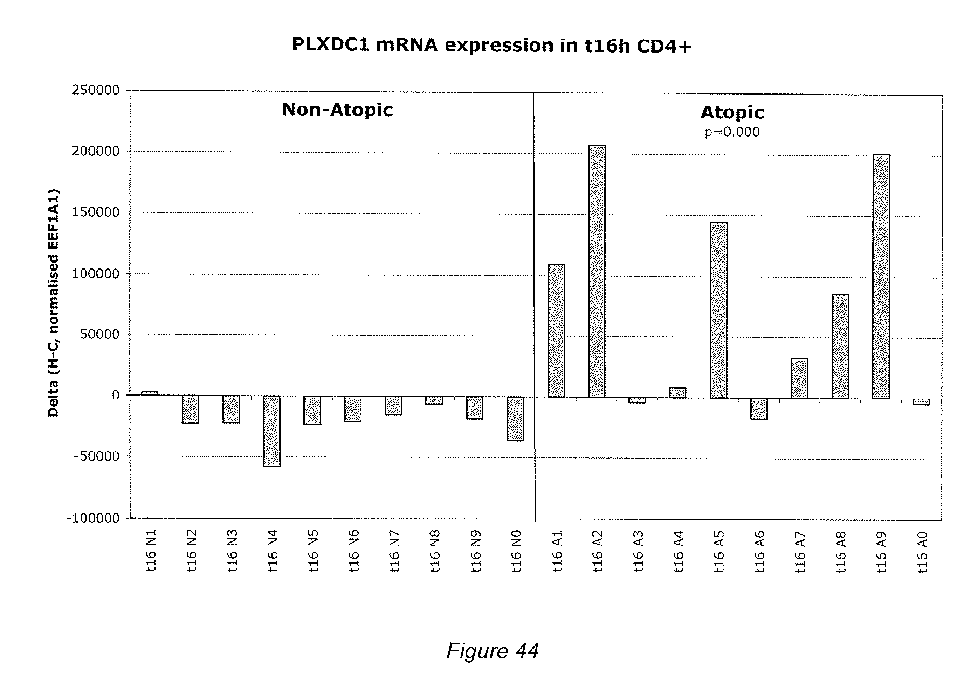

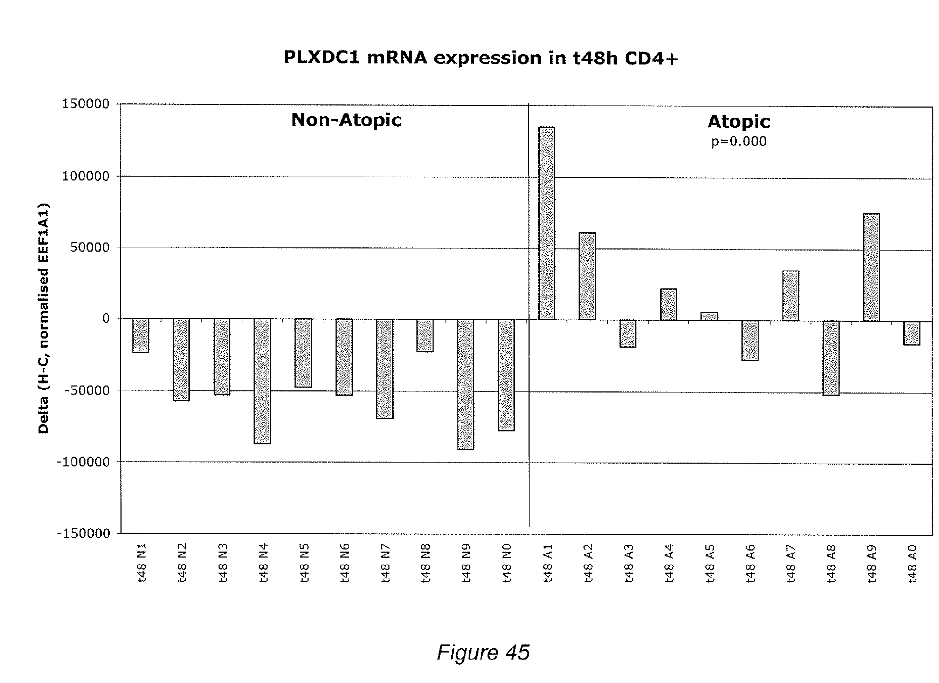

The data were normalised to the FEF1A1 housekeeping gene, and the results are shown in Figures 41 to 45. It can be seen from Figure 41 that 7 of the samples from putative allergic individuals initially selected for the culture experiment displayed vigorous IL-4 gene transcription, while 3 samples, indicated by arrows, showed low/undetectable responses. This indicated that, despite their positive skin-prick test status, the numbers of HDM-specific T-cells present in the circulation of these individuals at the time of sample were too low for detection of in vitro immune responses. These 3 subjects showed the lowest response in the skin prick test (5-6 mm), and also showed a low level of expression of DACT1 and PLXDC1, but a high level of expression of LAMP3.

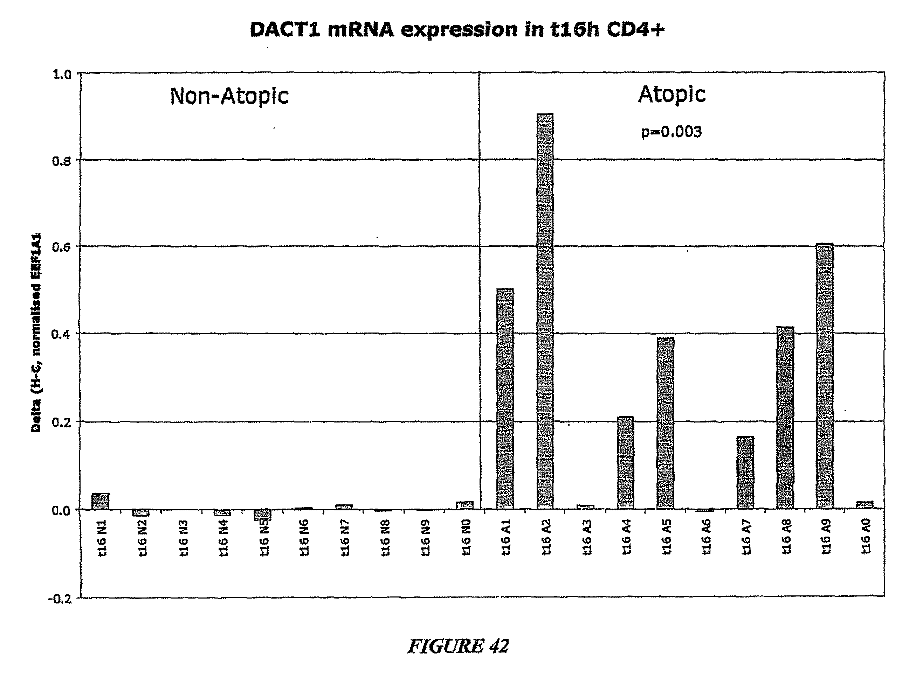

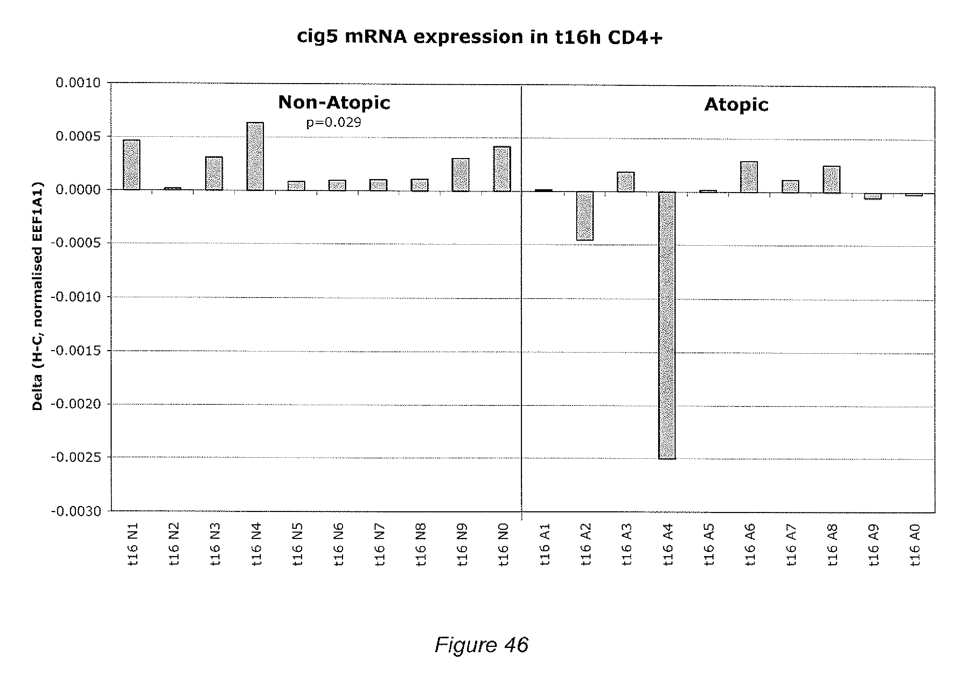

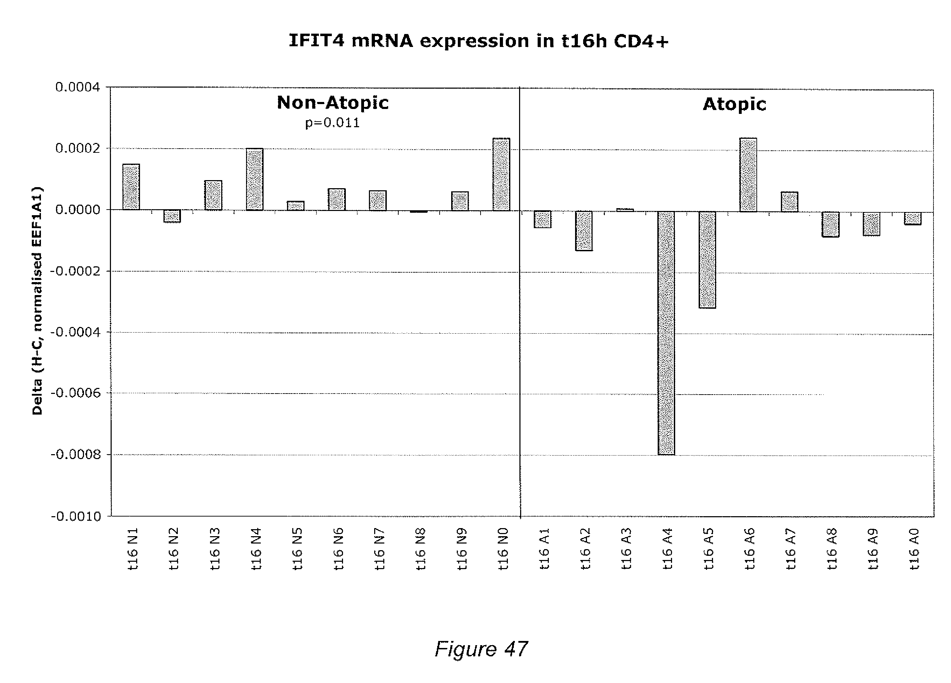

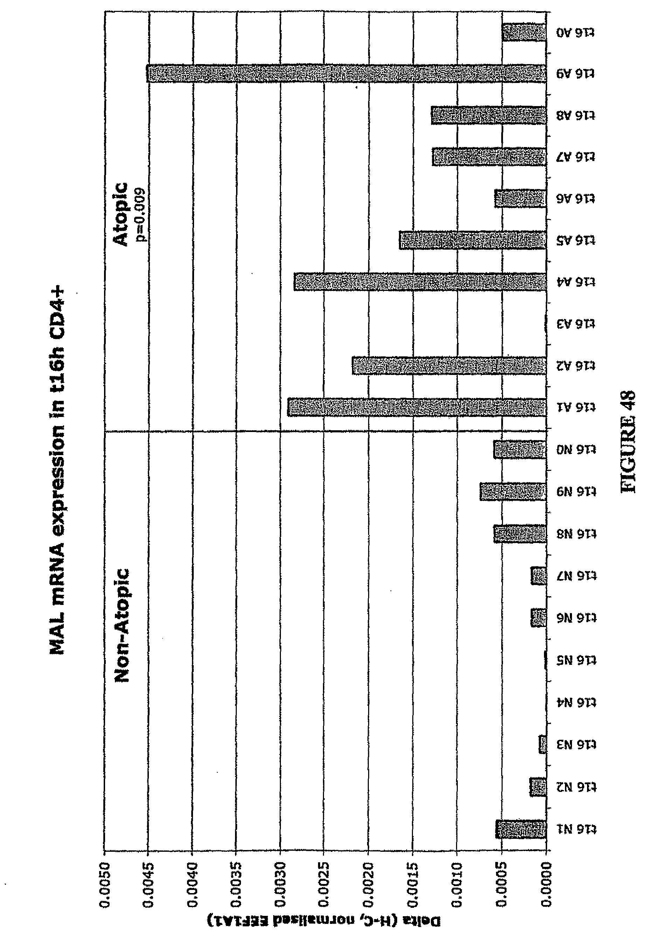

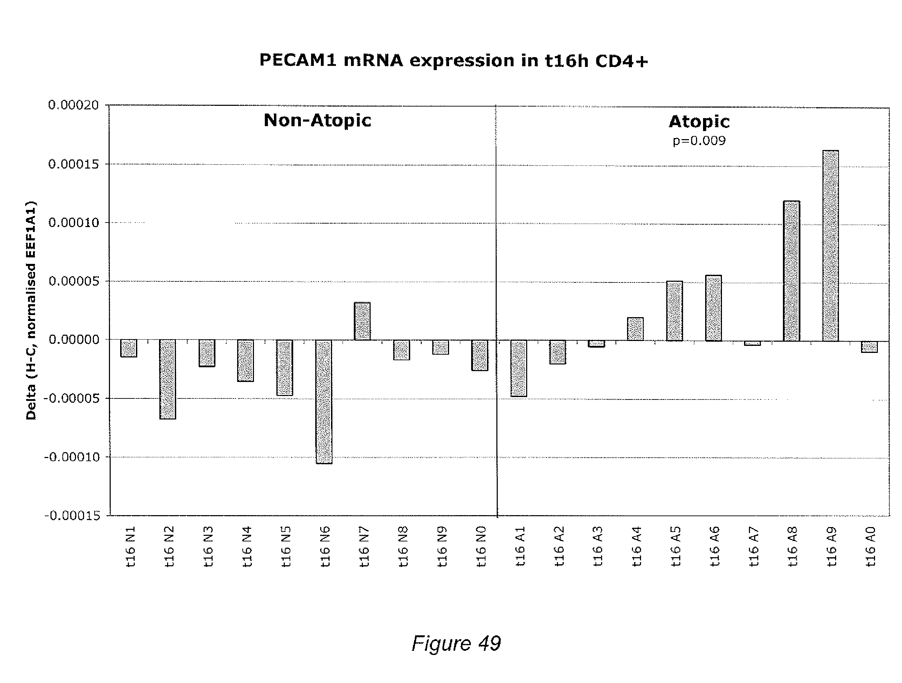

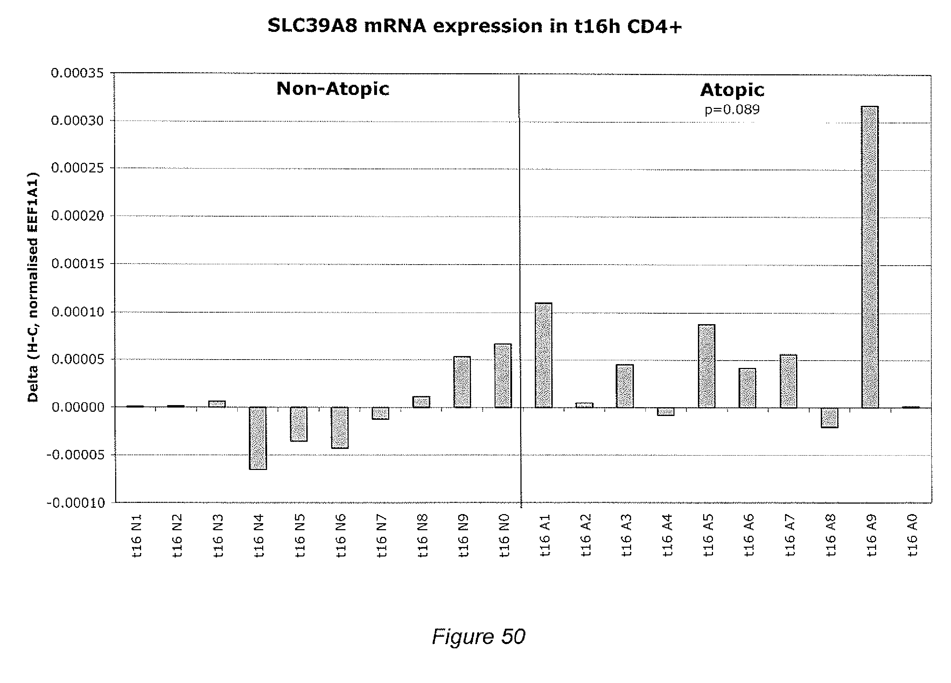

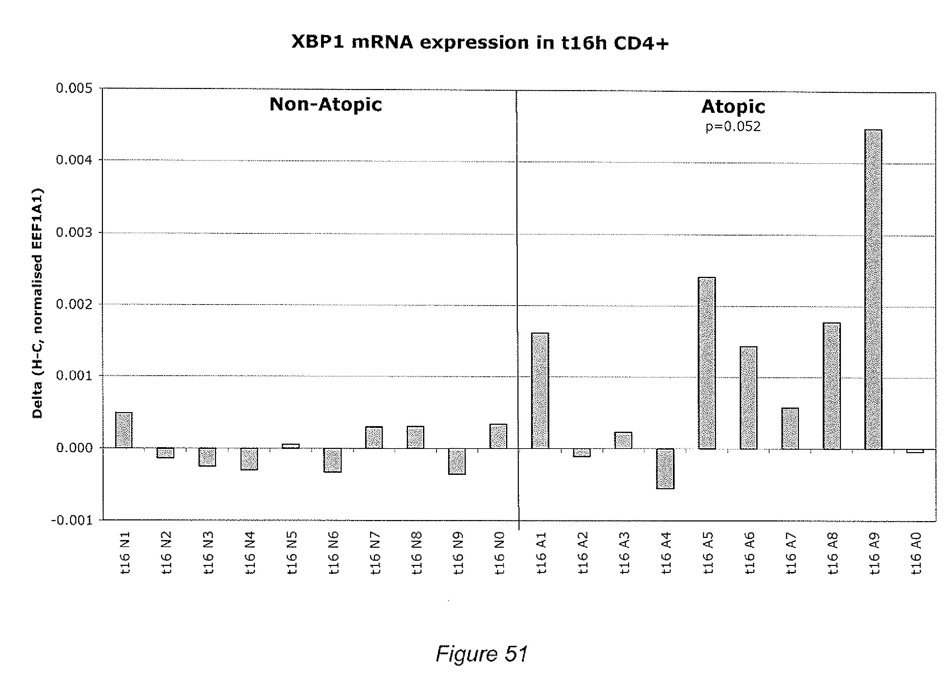

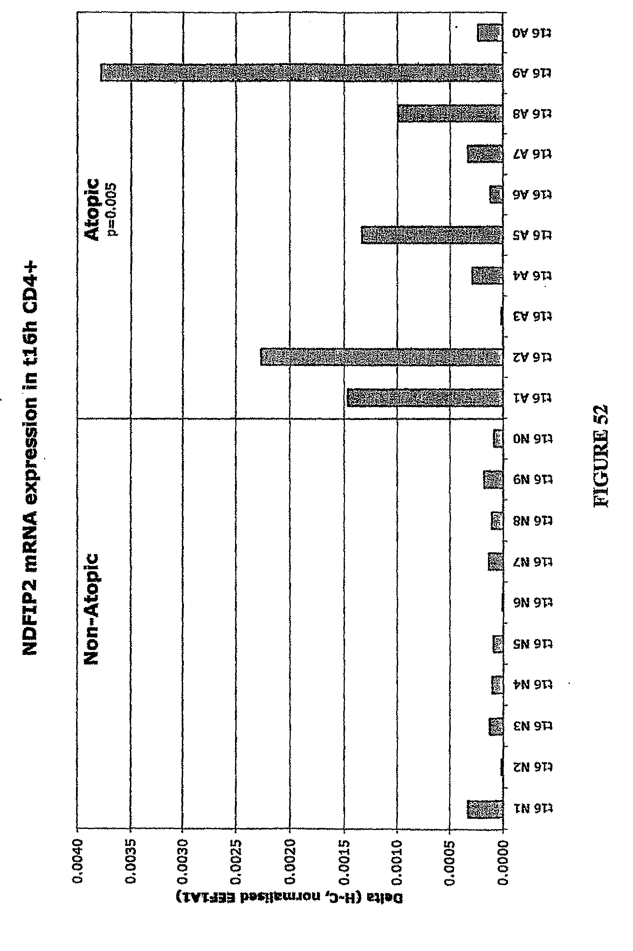

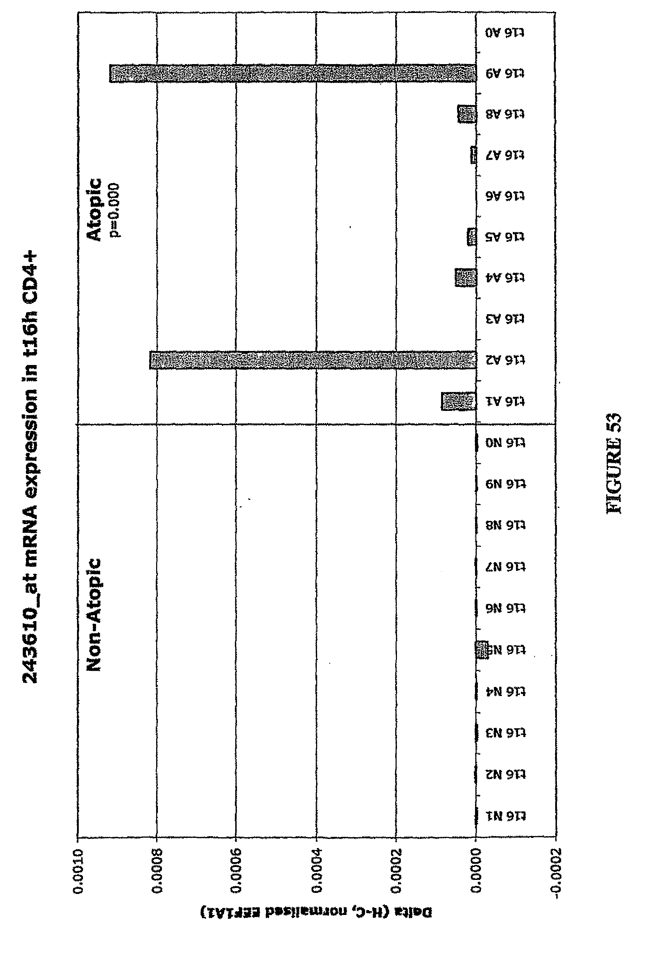

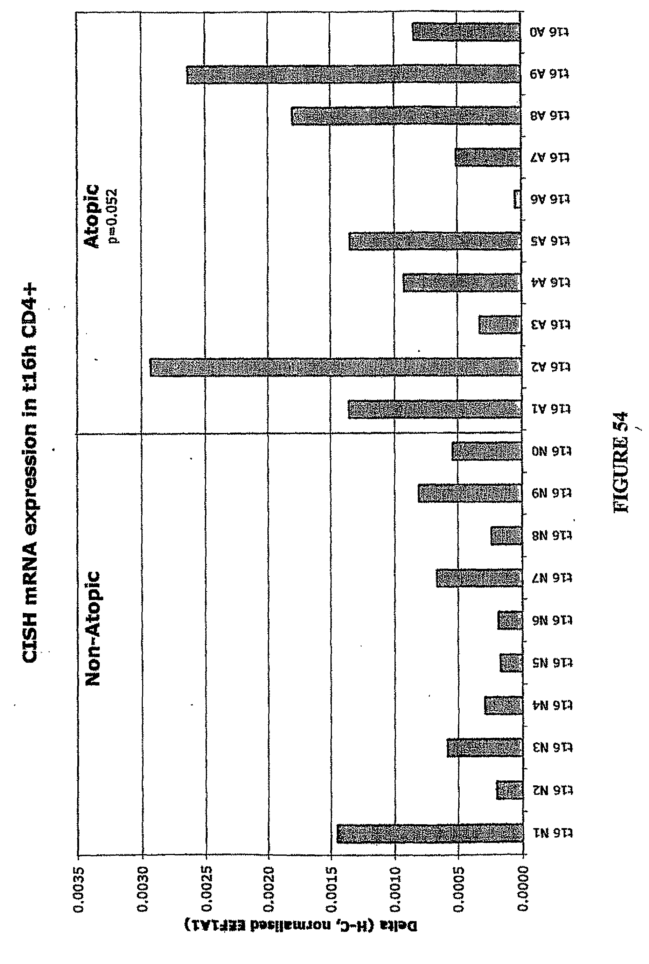

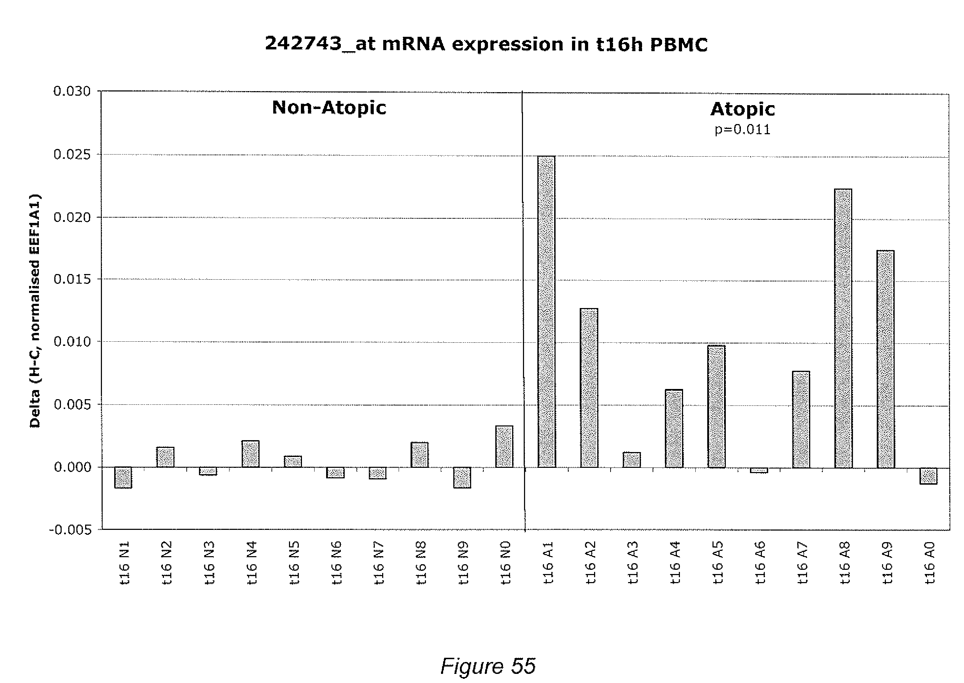

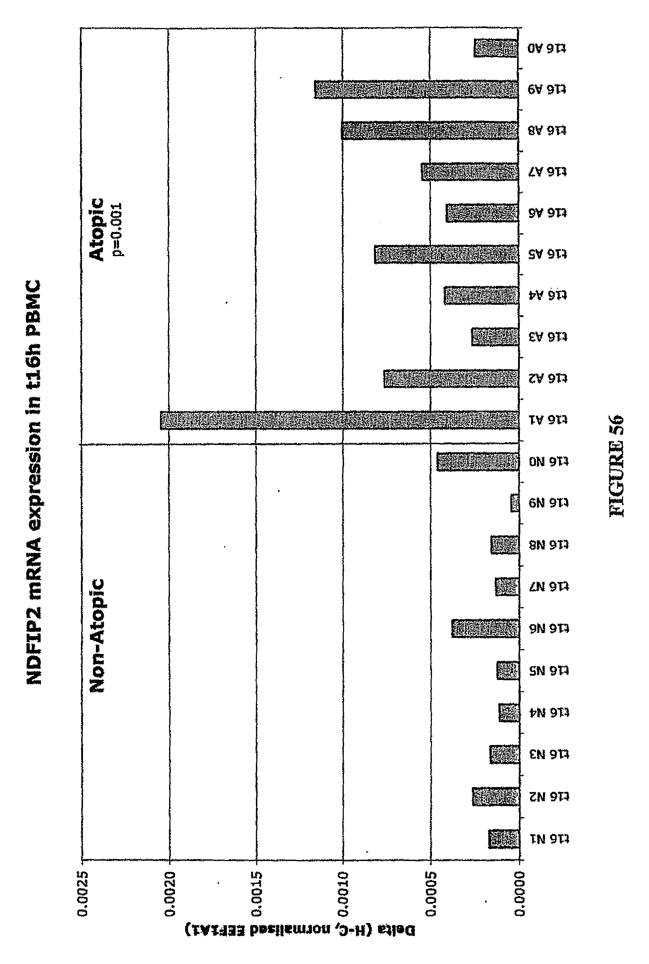

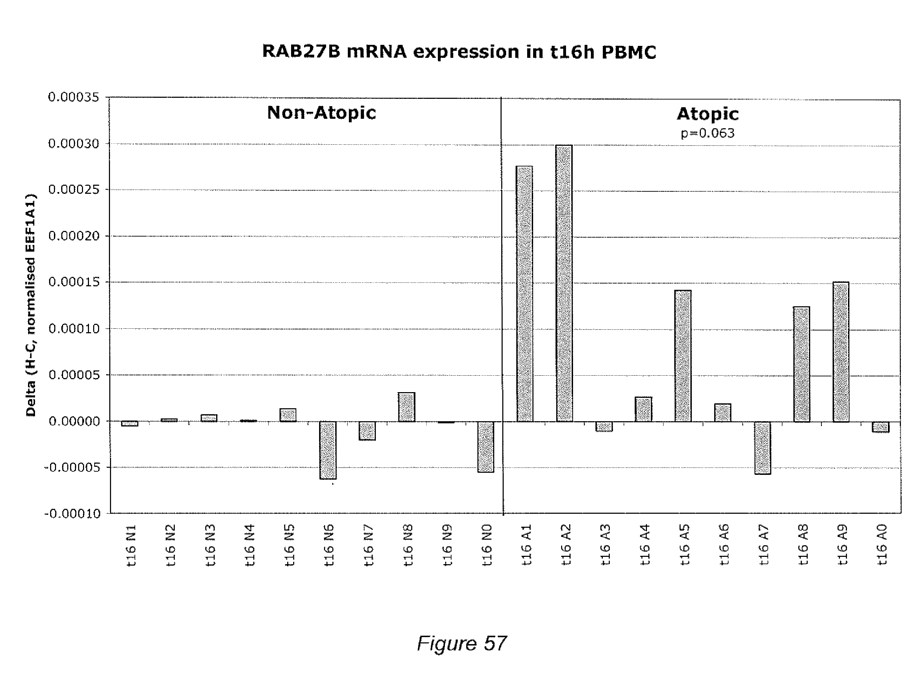

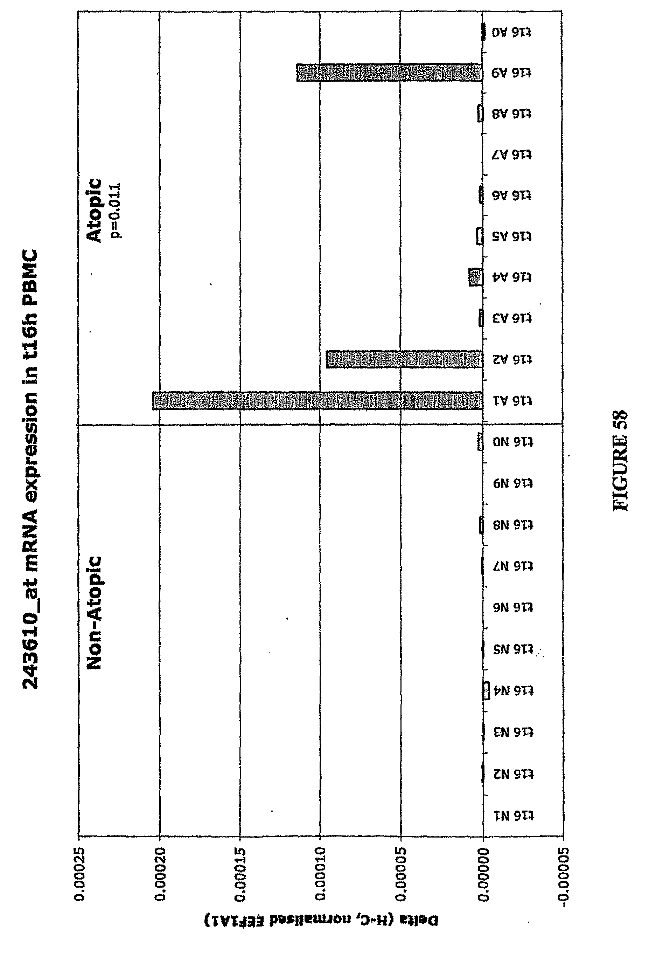

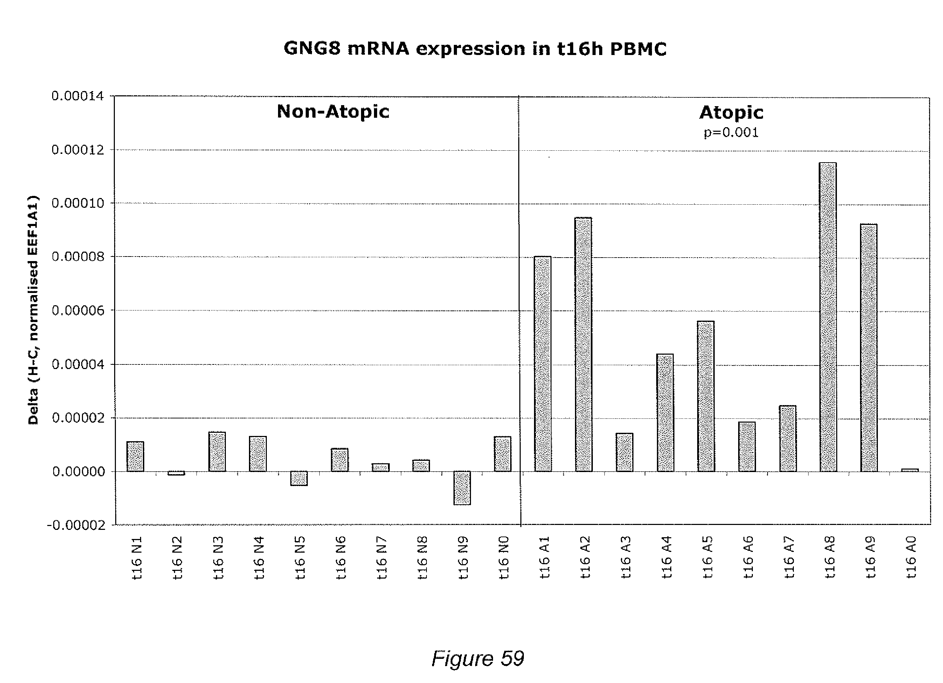

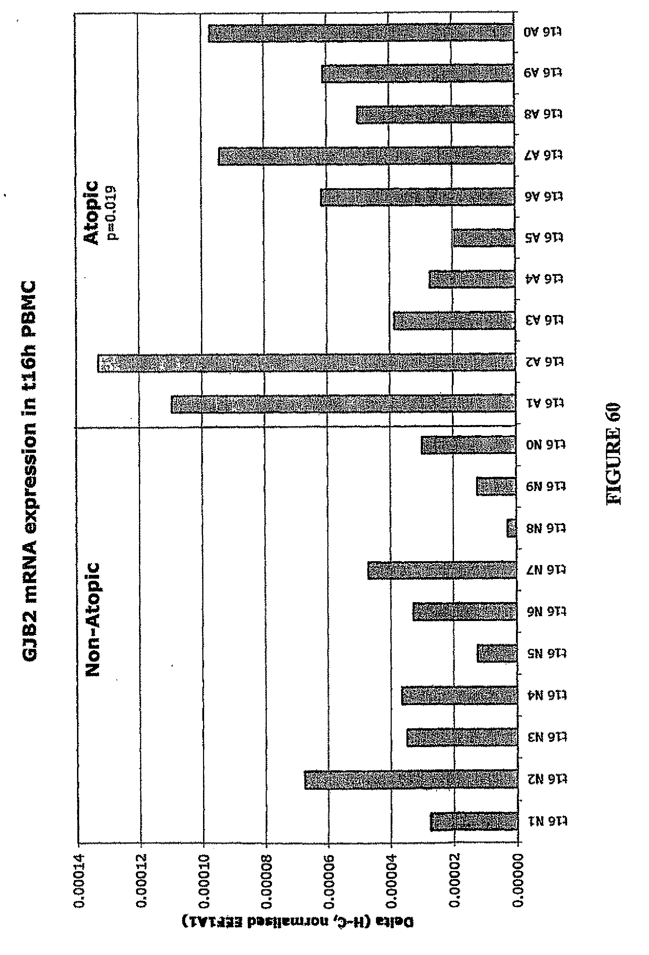

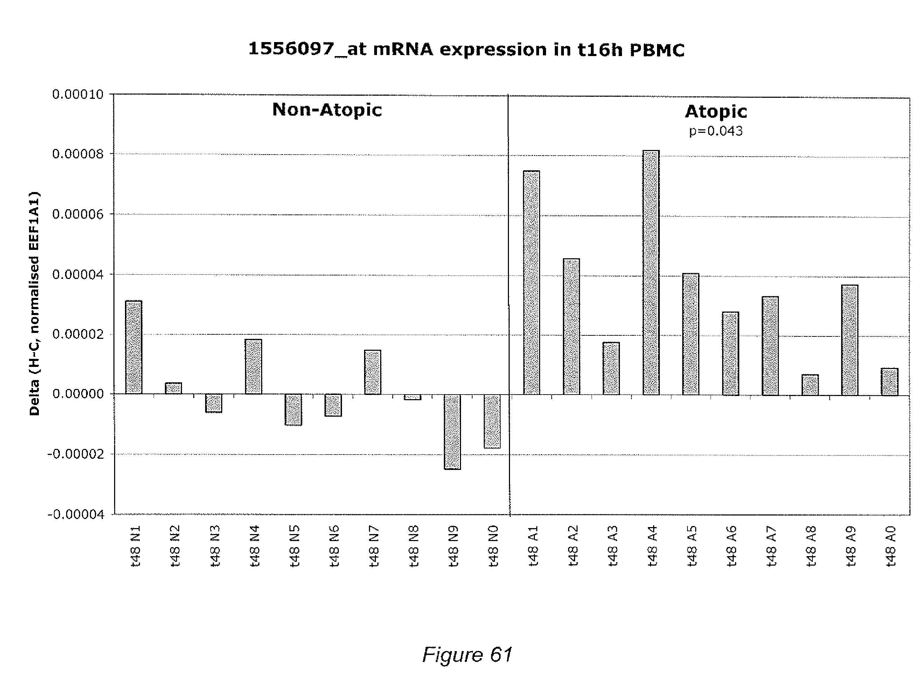

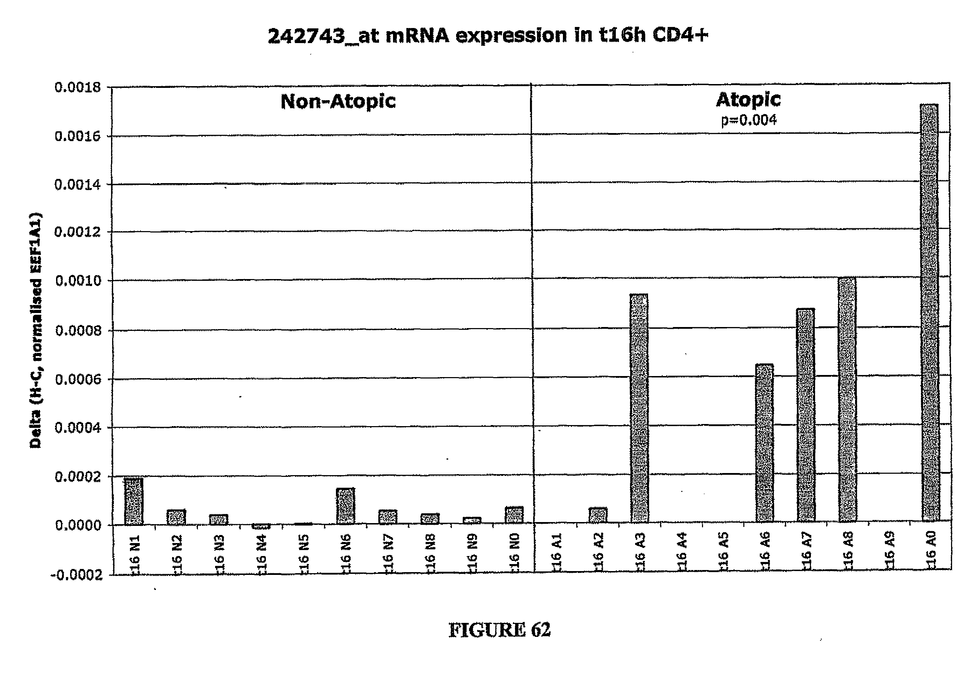

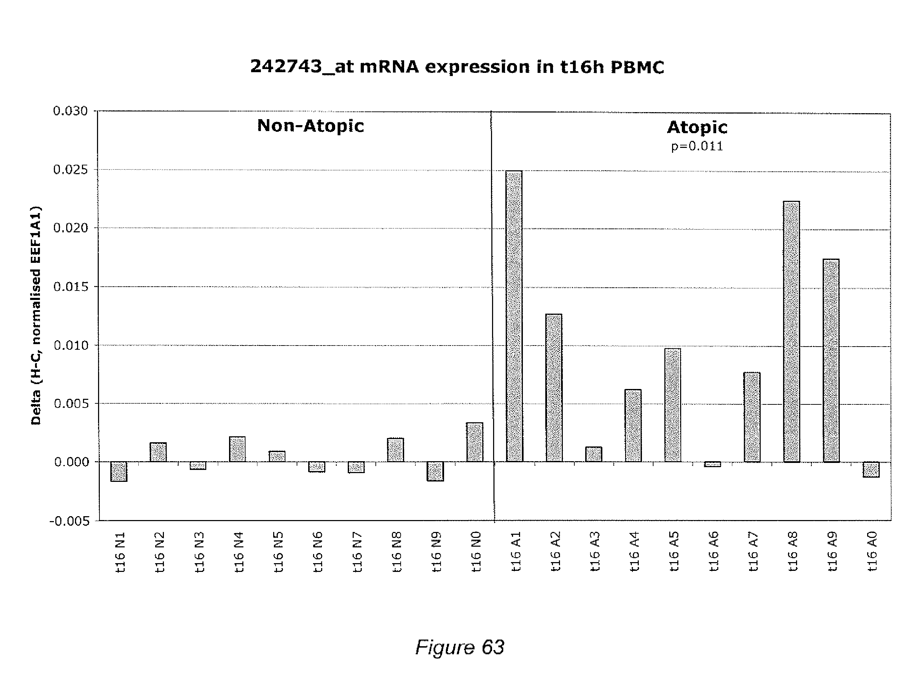

For the validation experiment, RNA from the individual samples employed to generate the pools used for microarray analysis of the PMBC or CD4 kinetic experiments at the 16 and 48hr time points was converted to cDNA, and then quantitative PCR was performed to detect a series of representative genes. The results are summarised in Figures 42 to 63. In some cases a significant change was seen only after 48hr incubation.

Validation is performed in the same way for the remaining genes, using the following primer sets:
IL17RB forward: 5'TGTGGAGGCACGAAAGGAT3' (SEQ ID NO:9)
   IL17RB reverse: GATGGGTAAACCACAAGAACCT3' (SEQ ID NO:10)
KRT1 forward: 5'TCAATCTCGGTTGGATTCGGA3' (SEQ ID NO:11)
   KRT1 reverse: 5'CTGCTTGGTAGAGTGCTGTAAGG3' (SEQ ID NO:12)
LNPEP forward: 5'TTCACCAATGATCGGCTTCAG3' (SEQ ID NO:13)
   LNPEP reverse: 5'CTCCATCTCATGCTCACCAAG3' (SEQ ID NO:14)
OAZ forward: 5'TCAATTTACACCTGCGATCACTG3' (SEQ ID NO:19)
   OAZ reverse: 5'GTTGTGGGTCGTCATCACCA3' (SEQ ID NO:20)
   and
RASGRP3 forward: 5'TCAGCCTCATCGACATATCCA3' (SEQ ID NO:25)
   RASGRP3 reverse: 5'TCAGCCAATTCAATGGGCTCC3' (SEQ ID NO:26)

It will be apparent to the person skilled in the art that while the invention has been described in some detail for the purposes of clarity and understanding, various modifications and alterations to the embodiments and methods described herein may be made without departing from the scope of the inventive concept disclosed in this specification.

### Recitals of the invention are as follows:

1. An isolated nucleic acid molecule comprising:
   (a) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or
   (b) an isolated nucleic acid molecule which is the complement of the nucleic acid of (a), or
   (c) an isolated nucleic acid molecule which hybridizes under stringent conditions to the nucleic acid of (a) or (b).
2. An isolated nucleic acid molecule according to claim 1,
   wherein the nucleic acid molecule is an allergy-associated gene.
3. An isolated nucleic acid molecule according to claim 2,
   wherein the allergy-associated gene is selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH or combinations thereof.
4. An isolated nucleic acid molecule according to claim 2,
   wherein the allergy-associated gene is KRT1, PECAM1 or PLXDC1.
5. An isolated nucleic acid molecule according to any one of claims 1 to 4, wherein the mammal is a human or a domestic, companion or zoo animal.
6. An isolated nucleic acid molecule selected from the group consisting of
   (a) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively,
   (b) an isolated nucleic acid molecule which is the complement of the nucleic acid of (a), and
   (c) an isolated nucleic acid molecule which hybridizes under stringent conditions to the nucleic acid of (a) or (b),
      for use in the therapy or prophylaxis of an allergic condition.
7. An isolated polypeptide molecule encoded by an isolated nucleic acid according to any one of claims 1 to 6.
8. An isolated polypeptide molecule encoded by a nucleic acid according to any one of claims 1 to 6, for use in the therapy or prophylaxis of an allergic condition.
9. A therapeutic or prophylactic composition comprising
   (i) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of cig5, IFIT4, LAMP3, DACT1, KRT1, LNPEP, NCOA3, OAZ, PECAM1, PLXDC1, RASGRP3, SLC39A8, RAB27B, GNG8 and GJB2, or in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or
   (ii) an isolated nucleic acid molecule which is the complement of the nucleic acid of (a),
   (iii) an isolated nucleic acid molecule which hybridizes under stringent conditions to the nucleic acid of (a) or (b), or
   (iv) an isolated polypeptide molecule encoded by the nucleic acid of (a), (b) or (c). together with a pharmaceutically acceptable carrier.
10. A therapeutic or prophylactic composition according to claim 9, wherein the nucleic acid molecule is an allergy-associated gene.
11. A therapeutic or prophylactic composition according to claim 10, wherein the allergy-associated gene is selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH or combinations thereof.
12. A therapeutic or prophylactic composition according to claim 10, wherein the allergy-associated gene is KRT1, PECAM1 or PLXDC1.
13. A therapeutic or prophylactic composition according to any one of claims 10 to 12, wherein the carrier is selected from the group consisting of sterile water, sodium phosphate, mannitol, sorbitol, sodium chloride, and any combination thereof.
14. An isolated nucleic acid molecule comprising:
   (a) an isolated nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the nucleic acid comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or
   (b) an isolated nucleic acid molecule which is the complement of the nucleic acid of (a), or
   (c) an isolated nucleic acid molecule which hybridizes under stringent conditions to the nucleic acid of (a) or (b); or
   (d) an isolated polypeptide molecule encoded by the nucleic acid of (a), (b) or (c), for use in the therapy or prophylaxis of an allergic condition.
15. A primer set for amplification of a nucleic acid molecule whose expression is modulated in a mammal suffering from or at elevated risk of developing an allergic condition, such that the level of expression of the nucleic acid differs from that in a mammal which is not suffering from or at elevated risk of developing the allergic condition, in which the primer set is selected from the group consisting of:
   cig5 forward: 5'CAAGACCGGGGAGAATACCTG3' (SEQ ID NO:1)
      cig5 reverse: 5'GCGAGAATGTCCAAATACTCACC3' (SEQ ID NO:2),
   IFIT4 forward: 5'GAGTGAGGTCACCAAGAATTC3' (SEQ ID NO:3)
      IFIT4 reverse: 5'CACTCTATCTTCTAGATCCCTTGAGA3' (SEQ ID NO:4),
   LAMP3 forward: 5'GCGTCCCTGGCCGTAATTT3' (SEQ ID NO:5)
      LAMP3 reverse: 5'TGGTTGCTTAGCTGGTTGCT3' (SEQ ID NO:6),
   DACT1 forward: 5'AACTCGGTGTTCAGTGAGTGT3' (SEQ ID NO:7)
      DACT1 reverse: 5'GGAGAGGGAACGGCAAACT3' (SEQ ID NO:8),
   IL17RB forward: 5' TGTGGAGGCACGAAAGGAT3' (SEQ ID NO:9)
      IL17RB reverse: GATGGGTAAACCACAAGAACCT3' (SEQ ID NO:10),
   KRT1 forward: 5'TCAATCTCGGTTGGATTCGGA3' (SEQ ID NO:11)
      KRT1 reverse: 5'CTGCTTGGTAGAGTGCTGTAAGG3' (SEQ ID NO:12),
   LNPEP forward: 5'TTCACCAATGATCGGCTTCAG3' (SEQ ID NO:13)
      LNPEP reverse: 5'CTCCATCTCATGCTCACCAAG3' (SEQ ID NO:14),
   MAL forward: 5'TCGTGGGTGCTGTGTTTACTCT3' (SEQ ID NO:15)
      MAL reverse: 5' CAGTTGGAGGTTAGACACAGCAA3' (SEQ ID NO:16),
   NCOA3 forward: 5'CCTGTCTCAGCCACGAGCTA3' (SEQ ID NO:17)
      NCOA3 reverse: 5'TCCTGAAAGATCATGTCTGGTAA3' (SEQ ID NO:18),
   OAZ forward: 5'TCAATTTACACCTGCGATCACTG3' (SEQ ID NO:19)
      OAZ reverse: 5'GTTGTGGGTCGTCATCACCA3' (SEQ ID NO:20),
   PECAM1 forward: 5'AGTCCAGATAGTCGTATGTGAAATGC3' (SEQ ID NO:21)
      PECAM1 reverse: GGTCTGTCCTTTTATGACCTCAAAC3' (SEQ ID NO:22),
   PLXDC1 forward: 5'CCTGGGCATGTGTCAGAGC3' (SEQ ID NO:23)
      PLXDC1 reverse: 5'GGTGTTGGAGAGTATTGTGTGG3' (SEQ ID NO:24),
   RASGRP3 forward: 5'TCAGCCTCATCGACATATCCA3' (SEQ ID NO:25)
      RASGRP3 reverse: 5'TCAGCCAATTCAATGGGCTCC3' (SEQ ID NO:26),
   SLC39A8 forward: 5'GCAGTCTTACAGCAATTGAACTTT3' (SEQ ID NO:27)
      SLC39A8 reverse: 5'CCATATCCCCAAACTTCTGAA3' (SEQ ID NO:28),
   XBP1 forward: 5'GTAGATTTAGAAGAAGAGAACCAAAAAC3' (SEQ ID NO:29)
      XBP1 reverse: 5'CCCAAGCGCTGTCTTAACTC3' (SEQ ID NO:30),
   NDFIP2 forward: 5'AGTGGGGAATGATGGCATTTT3' (SEQ ID NO:31)
      NDFIP2 reverse: AAATCCGCAGATAGCACCA3' (SEQ ID NO:32),
   RAB27B forward: 5'CAGAAACTGGATGAGCCAACT3' (SEQ ID NO:33)
      RAB27B reverse: 5'GACTTCCCTCTGATCTGGTAGG3' (SEQ ID NO:34),
   243610_at forward: 5'TGCATTGACAACGTACTCAGAA3' (SEQ ID NO:35)
      243610_at reverse: 5'TCATCTTGACAGGGATAAGCAT3' (SEQ ID NO:36),
   GNG8 forward: 5'GAACATCGACCGCATGAAGGT3' (SEQ ID NO:37)
      GNG8 reverse: 5'AGAACACAAAAGAGGCGCTTG3' (SEQ ID NO:38),
   GJB2 forward: 5'GCTTCCTCCCGACGCAGA3' (SEQ ID NO:39)
      GJB2 reverse: 5'AACGAGGATCATAATGCGAAA3' (SEQ ID NO:40),
   1556097_at forward: 5'TCTTATTTCACTTTCTCAACTCATCA3' (SEQ ID NO:41)
      1556097_at reverse: 5'GGCATAACCTGAATGTATAATTCAA3' (SEQ ID NO:42), 242743_at forward: 5'GAAAAAGCTGTTGAGTGAAGAAGACT3' (SEQ ID NO:43) 242743_at reverse: 5'TGCAGGATGAGCAATGCTGAGA3' (SEQ ID NO:44),
      and
   CISH forward: 5'GGGAATCTGGCTGGTATTGG3' (SEQ ID NO:45)
      CISH reverse: 5'TTCTGGCATCTTCTGCAGGTGTT3' (SEQ ID NO:46).
16. A kit for screening for an agent capable of treating or preventing an allergic disorder in a mammal, comprising one or more primers specific for a region of at least one gene which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively.
17. A kit according to claim 16, in which the primer sets are as defined in claim 15.
18. A method of treating or preventing an allergic disorder in a mammal, comprising the step of administering to the mammal a therapeutic or prophylactic agent in an amount sufficient to regulate the expression of a gene which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or a combination of two or more of these genes.
19. A method according to claim 18, in which the agent is a down-regulator of expression of one or more genes selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or a combination of two or more of these genes.
20. A method according to claim 19, in which the gene is KRT₁, PECAM₁ or PLXDC₁.
21. A method according to claim 18, in which the agent is an up-regulator of expression of cig5, IFIT4, or LAMP3.
22. A method of screening for an agent capable of treating or preventing an allergic disorder in a mammal, the method comprising:
   (a) providing a biological sample from a mammal;
   (b) determining the degree of activation of one or more specific genes in the sample,
      wherein the gene is associated with an allergic disorder;
   (c) contacting the biological sample with a candidate agent; and
   (d) determining whether the degree of activation changes in the sample following the contacting step,
   wherein a change in the degree of activation observed in the presence of the agent compared to that observed in the absence of the agent indicates that the agent is capable of treating or preventing an allergic disorder.
23. A method of screening for an agent capable of treating or preventing an allergic disorder in a mammal, the method comprising:
   (a) providing a biological sample from the mammal;
   (b) determining the level of mRNA transcripts from one or more allergy-associated genes,
      in which the gene is selected from the group consisting of cig5, IFIT4, LAMP3, DACT1, IL17RB, KRT1, LNPEP, MAL, NCOA3, OAZ, PECAM1, PLXOC1, RASGRP3, SLC39A8, XBP1, NDFIP2, RAB27B, GNG8, GJB2 and CISH, or comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively;
   (c) contacting the biological sample with the agent; and
   (d) determining whether the level of mRNA transcripts changes in the sample following the contacting step,
   in which the change in the level of mRNA transcripts in the sample following contact with the agent compared to in the absence of the agent indicates that the agent is capable of treating or preventing an allergic disorder.
24. A method of selecting an appropriate therapeutic agent for treatment of a mammal which suffers from or is predisposed to developing an allergic disorder, comprising the steps of:
   (a) obtaining a biological sample from the mammal;
   (b) determining the level in the sample of mRNA transcripts from one or more genes which is selected from the group consisting of cig5, IFIT4, LAMP3, DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, or combination thereof; and
   (c) selecting a therapeutic modulator which compensates for the presence or absence of the mRNA transcript or protein.
25. A method according to any one of claims 22 to 24, in which the allergy-associated gene is upregulated in allergen-challenged PBMC from atopic individuals but is upregulated weakly if at all in PBMC from individuals who are not allergic to that allergen.
26. A method according to claim 25, in which the gene is one or more selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 and CISH, and genes which comprise a sequence selected from the group consisting of sequences identified by probes 243610_at on human chromosome 9q21.13 at locus 138255, 1556097_at on human chromosome 15q25.2 and 242743_at on human chromosome 16p12.1 respectively.
27. A method according to claim 26, in which the gene is upregulated in atopic individuals and down-regulated in non-atopic individuals.
28. A method according to claim 27, in which the gene is KRT₁, PECAM₁ or PLXDC₁.
29. A method according to any one of claims 22 to 24, in which the allergy-associated gene is down-regulated in allergen-challenged PBMC from non-atopic individuals, but is not down-regulated in PBMC from atopic individuals.
30. A method according to claim 29, in which the gene is selected from the group consisting of cig5, IFIT4 and LAMP3.
31. A method according to any one of claims 18 to 21, wherein the gene is associated with an allergic disorder.
32. A method according to claim 23 or claim 24, wherein the mRNA is detected by reverse transcription polymerase chain reaction (RT-PCR), or using specific nucleic acid arrays utilising microchip technology.
33. A method according to any one of claims 22 to 30, wherein the biological sample is isolated from an atopic mammal.
34. A method according to any one of claims 22 to 30, wherein the biological sample is isolated from a non-atopic mammal.
35. A method according to any one of claims 22 to 34, wherein the biological sample is selected from the group consisting of blood, bone marrow, plasma, serum, lymph, cerebrospinal fluid, or a cellular or fluid component thereof; external sections of the skin, respiratory, intestinal, and genitourinary tracts; other secretions such as tears, saliva, or milk; tissue or organ biopsy samples; or cultured cells or cell culture supernatants.
36. A method according to any one of claims 22 to 34, wherein the biological sample is blood or lymph, or a cellular or fluid component thereof.
37. A method according to any one of claims 22 to 34, wherein the biological sample is bone marrow-derived mononuclear cells from peripheral blood (PBMC), which have been stimulated by *in vitro* exposure to one or more allergens to which the mammal is allergic.
38. A method according to any one of claims 18 to 20, wherein the agent is an siRNA molecule or an anti-sense oligonucleotide.
39. A method according to claim 38, wherein the agent is an anti-sense oligonucleotide of 8 to 50 nucleotide bases in length, which specifically hybridizes with a coding region of a gene which is selected from the group consisting of DACT1, IL17RB, KRT1, LNPEP, MAL, NCOA3, OAZ, PECAM1, PLXDC1, RASGRP3, SLC39A8, XBP1, NDFIP2, RAB27B, GNG8, GJB2 or CISH, or which comprises a sequence selected from the group consisting of sequences identified by probes 243610_at on human chromosome 9q21.13 at locus 138255, 1556097_at on human chromosome 15q25.2 and 242743_at on human chromosome 16p12.1 respectively, and inhibits the expression of the gene.
40. A method according to any one of claims 18 to 20, wherein the agent is either a polyclonal or monoclonal antibody.
41. A method according to claim 40, wherein the antibody is a humanized monoclonal antibody.
42. A method according to any one of claims 18 to 20, wherein the agent is a steroid, a β-2 agonist, a methylxanthine, a leukotriene modifier, an anti-cholinergic, a systemic corticosteroid, or an anti-histamine.

## Claims

1. A method of screening for an agent capable of treating or preventing an allergic disorder in a mammal, the method comprising:
(a) providing a biological sample from the mammal which is suspected of, or susceptible to the development of an allergic disorder;
(b) exposing the biological sample to an environmental allergen;
(c) obtaining RNA from the biological sample in step (b);
(d) determining the level of activation of NDFIP2;
(e) providing a further biological sample from said mammal and contacting said sample with a test agent and then repeating steps (b) to (d), wherein any change in the level of activation of NDFIP2 following the contacting step indicates that the agent is capable of treating or preventing an allergic disorder.

2. A method according to claim 1, wherein step (d) further comprises determining the level of activation of one or more genes selected from the group consisting of cig5, IFIT4, LAMP3, DACT1, IL17RB, KRT1, LNPEP, MAL, NC0A3, OAZ, PECAM1, PLXDC1, RASGRP3, SLC39A8, XBP1, RAB27B, GNG8, GJB2 and CISH.

3. A method according to any preceding claim, wherein the mammal is a human.

4. A method according to any preceding claim, wherein the biological sample is selected from the group consisting of blood, bone marrow, plasma, serum, lymph, cerebrospinal fluid, or a cellular or fluid component thereof; external sections of the skin, respiratory, intestinal, and genitourinary tracts; other secretions such as tears, saliva, or milk; tissue or organ biopsy samples; or cultured cells or cell culture supernatants.

5. A method according to any preceding claim, wherein the biological sample is blood or lymph, or a cellular or fluid component thereof.

6. A method according to any preceding claim, wherein the biological sample is bone marrow-derived mononuclear cells from peripheral blood (PBMC), which have been stimulated by in vitro exposure to one or more allergens to which the mammal is allergic.

7. Use of an agent identified by the method of any one of claims 1 to 6 in the manufacture of a medicament used to modulate the activation of NDFIP2.

8. Use according to claim 7, in which the agent is a down-regulator of the expression NDFIP2.

9. Use according to claim 7 or 8, wherein the agent is an siRNA molecule or an anti-sense oligonucleotide.

10. Use according to claim 7 or 8, wherein the agent is either a polyclonal or monoclonal antibody.

11. Use according to claim 7 or 8, wherein the agent is a steroid, a β 2 agonist, a methylxanthine, a leukotriene modifier, an anti-cholinergic, a systemic corticosteroid, or an anti-histamine.

12. A kit for screening for an agent capable of treating or preventing an allergic disorder in a mammal, comprising one or more primers specific for a region of NDFIP2.
